# EUROPEAN PATENT APPLICATION

(11) **EP 4 293 102 A1**
(43) Date of publication of application: **20.12.2023**
(21) Application number: 22780924.1
(22) Date of filing: 29.03.2022
(51) Int. Cl.: C12M 1/00, C12M 3/00, C12N 5/071

(54) **CELL MASS-FORMING MEMBER, CULTURE CONTAINER, METHOD FOR PRODUCING CULTURED CELLS, AND CULTURED CELLS WITH CELL MASS-FORMING MEMBER**

(30) Priority: 30.03.2021 JP 2021058013
(71) Applicant: National University Corporation Hokkaido University, Sapporo-shi, Hokkaido 060-0808 (JP); Oji Holdings Corporation, Chuo-ku Tokyo 104-0061 (JP)
(72) Inventor: MIYATAKE Yukiko, Sapporo-shi, Hokkaido 060-0808 (JP); SHIGETOMI Kaori, Sapporo-shi, Hokkaido 060-0808 (JP); TOKUNO Hisako, Tokyo 104-0061 (JP); DAI Kotaro, Tokyo 104-0061 (JP); SHINOTSUKA Kei, Tokyo 104-0061 (JP)
(74) Representative: Zacco GmbH
(86) International application number: PCT/JP2022/015460
(87) International publication number: WO 2022/210713

(57) **Abstract**

According to one embodiment of the present invention, provided are: a cell mass-forming member that is capable of easily forming a cell mass and superior in industrial mass productivity; a culture container equipped with the cell mass-forming member; a method for producing cultured cells using the cell mass-forming member; and cultured cells with a cell mass-forming member that are equipped with the cell mass-forming member. The cell mass-forming member 1B according to one example of the present invention has a base material 2, wherein: an adhesion inhibition area 3A and a cell adhesion area 4B are formed on the surface 2a of the base material 2; a micro-concavo-convex structure area 6 including a plurality of convex portions 7 is formed in the cell adhesion area 4B; and a hydrophilic coating layer 5 is formed on both the adhesion inhibition area 3A and the cell adhesion area 4B. The culture container and the cultured cells with a cell mass-forming member according to one example of the present invention are equipped with the cell mass-forming member 1B. The method for producing cultured cells according to one example of the present invention comprises using the cell mass-forming member 1B.

## Description

### Technical Field

The present invention relates to a cell mass-forming member, a culture vessel, a method for producing cultured cells, and cultured cells with a cell mass-forming member.

The present application claims priority from the Japanese patent application No. 2021-058013 filed on March 30, 2021, the contents of which are incorporated herein by reference.

### Background Art

In recent years, cell culture techniques have been utilized in various industrial fields (e.g., Patent Documents 1 and 2). Patent Document 1 proposes a cell culture vessel that can be suitably used in biochemical experiments, clinical experiments, drug development research, and the like.

Patent Document 2 proposes a cell culture substrate for forming a cell mass with morphological polarity and tissue movement characteristics, such as those observed in cancer tissues in vivo.

### Citation List

### Patent Document

Patent Document 1: WO 2007/125894
Patent Document 2: WO 2018/182044

### Summary of Invention

### Technical Problem

The bottom surface of the well of the cell culture vessel of Patent Document 1 is provided with a hydrophilic coating on which dot-like holes are patterned. This hydrophilic coating prevents non-specific adhesion of cells, and the cells adhere to the dot-like hole part. However, the dot-like holes are dispersedly arranged in the well as a result of patterning, and thus the cells cannot be cultured to form a cell mass.

In the cell culture substrate of Patent Document 2, cells are cultured on a rough surface portion not covered with a biocompatible polymer layer to form a cell mass. However, a process of selectively applying the biocompatible polymer uses a photolithography technique and is not suitable for mass production, thus leaving room for improvement in industrial mass productivity at the time of production.

The present invention provides a cell mass-forming member that can easily form a cell mass and has excellent industrial mass productivity; a culture vessel including the cell mass-forming member; a method for producing cultured cells using the cell mass-forming member; and cultured cells with the cell mass-forming member including the cell mass-forming member.

### Solution to Problem

The present invention has the following aspects.
(1) A cell mass-forming member including a substrate, wherein
   on a surface of the substrate, at least one or more attachment-preventing regions and at least one or more cell attachment regions are formed,
   in at least a part of the at least one or more cell attachment regions, a fine uneven structure region including a plurality of protrusions or a plurality of recesses is formed, and
   in both the at least one or more attachment-preventing regions and the at least one or more cell attachment regions a hydrophilic coating layer is formed.
(2) A cell mass-forming member including a substrate, wherein
   on a surface of the substrate, at least one or more attachment-preventing regions and at least one or more cell attachment regions are formed,
   in at least a part of the at least one or more cell attachment regions, a fine uneven structure region including a plurality of protrusions or a plurality of recesses is formed, and
   in either the at least one or more attachment-preventing regions or the at least one or more cell attachment regions, a hydrophilic coating layer is formed.
(3) The cell mass-forming member according to (1) or (2), wherein an average height of the plurality of protrusions or an average depth of the plurality of recesses is 250 nm or greater.
(4) The cell mass-forming member according to any of (1) to (3), wherein an aspect ratio of the average height of the plurality of protrusions to an average diameter of the plurality of protrusions or an aspect ratio of the average depth of the plurality of recesses to an average diameter of the plurality of recesses is from 0.5 to 5.0.
(5) The cell mass-forming member according to any of (1) to (4), wherein an average pitch between the plurality of protrusions or between the plurality of recesses is from 50 nm to 1 µm.
(6) The cell mass-forming member according to any of (1) to (5), wherein the at least one or more cell attachment regions are located at a position with a height difference relative to the at least one or more attachment-preventing regions in a thickness direction of the substrate.
(7) The cell mass-forming member according to any of (1) to (5), wherein a height difference between the at least one or more cell attachment regions and the surface of the substrate is from 0 to 3 µm.
(8) The cell mass-forming member according to any of (1) to (7), wherein a hydrophilic layer is formed in recesses between the plurality of protrusions of the fine uneven structure region or in bottoms of recesses between the plurality of protrusions of the fine uneven structure region.
(9) The cell mass-forming member according to any of (1) to (8), wherein the substrate contains at least one or more resins selected from the group consisting of polyethylene terephthalate), triacetyl cellulose, polycarbonate, cycloolefin polymers, cycloolefin copolymers, acrylic resins, polystyrene, and dimethylpolysiloxane.
(10) The cell mass-forming member according to any of (1) to (9), wherein a ratio of a major axis of an ellipse with a minimum area circumscribing the at least one or more cell attachment regions to a minor axis of the ellipse is from 1 to 3.
(11) The cell mass-forming member according to any of (1) to (10), wherein
   in a plan view of the cell mass-forming member, the at least one or more cell attachment regions are formed in an annular region surrounded by an outer diameter circle and an inner diameter circle, the outer-diameter circle having a diameter from 10 to 100 µm, a difference between the diameter of the outer diameter circle and a diameter of the inner diameter circle being from 200 nm to 30 µm, and in at least a part of the annular region, a fine uneven structure including a plurality of protrusions or a plurality of recesses being formed, and
   in both the at least one or more attachment-preventing regions and the at least one or more cell attachment regions, a hydrophilic coating layer is formed.
(12) A culture vessel including the cell mass-forming member of any of (1) to (11).
(13) A method for producing cultured cells, the method including culturing cells using the cell mass-forming member of any of (1) to (11), and then detaching the cultured cells from the cell mass-forming member.
(14) Cultured cells with a cell mass-forming member including:
   the cell mass-forming member of any of (1) to (11); and
   cultured cells attached to the cell mass-forming member.
(15) The cultured cells with a cell mass-forming member according to (14), wherein a cell mass including the cells forms a three-dimensional structure.

### Advantageous Effects of Invention

According to the present invention, there are provided the cell mass-forming member that can easily form a cell mass and has excellent industrial mass productivity; the culture vessel including the cell mass-forming member; the method for producing cultured cells using the cell mass-forming member; and the cultured cells with the cell mass-forming member including the cell mass-forming member.

### Brief Description of Drawings

FIG. 1 is a plan view schematically illustrating a cell mass-forming member according to an embodiment.
FIG. 2 is a plan view schematically illustrating a cell mass-forming member according to an embodiment.
FIG. 3 is an enlarged plan view schematically illustrating an example of a cell mass-forming member.
FIG. 4 is a cross-sectional view taken along line IV-IV of FIG. 3.
FIG. 5 is an enlarged plan view schematically illustrating an example of a cell mass-forming member.
FIG. 6 is a cross-sectional view taken along line VI-VI of FIG. 5.
FIG. 7 is an enlarged plan view schematically illustrating an example of a cell mass-forming member.
FIG. 8 is a cross-sectional view taken along line VIII-VIII of FIG. 7.
FIG. 9 is an enlarged plan view schematically illustrating an example of a cell mass-forming member.
FIG. 10 is a cross-sectional view taken along line X-X of FIG. 9.
FIG. 11 is an enlarged plan view schematically illustrating an example of a cell mass-forming member.
FIG. 12 is a cross-sectional view taken along line XII-XII of FIG. 11.
FIG. 13 is an enlarged plan view schematically illustrating an example of a cell mass-forming member.
FIG. 14 is a cross-sectional view taken along line XIV-XIV of FIG. 13.
FIG. 15 is an enlarged plan view schematically illustrating an example of a cell mass-forming member.
FIG. 16 is a cross-sectional view taken along line XVI-XVI of FIG. 15.
FIG. 17 is an enlarged plan view schematically illustrating an example of a cell mass-forming member.
FIG. 18 is a cross-sectional view taken along line XVIII-XVIII of FIG. 17.
FIG. 19 is an enlarged plan view schematically illustrating an example of a cell mass-forming member.
FIG. 20 is a cross-sectional view taken along line XX-XX of FIG. 19.
FIG. 21 is an enlarged plan view schematically illustrating an example of a cell mass-forming member.
FIG. 22 is a cross-sectional view taken along line XXII-XXII of FIG. 21.
FIG. 23 is an enlarged plan view schematically illustrating an example of a cell mass-forming member.
FIG. 24 is a cross-sectional view taken along line XXIV-XXIV of FIG. 23.
FIG. 25 is an enlarged plan view schematically illustrating an example of a cell mass-forming member.
FIG. 26 is a cross-sectional view taken along line XXVI-XXVI of FIG. 25.
FIG. 27 is a DIC observation image showing a result of culturing a human pancreatic ductal adenocarcinoma cell line PCI-55 after applying an MPC polymer to a cell mass-forming member of Example 1.
FIG. 28 is a DIC observation image showing a result of culturing a human pancreatic ductal adenocarcinoma cell line PCI-55 after applying an MPC polymer to a cell mass-forming member of Example 2.
FIG. 29 is a DIC observation image showing a result of culturing a mouse myoblast C2C12 line using the cell mass-forming member of Example 1.
FIG. 30 is a cross-sectional view schematically illustrating a cell mass-forming member according to a modified example of FIG. 3.
FIG. 31 is a cross-sectional view schematically illustrating a cell mass-forming member according to a modified example of FIG. 3.
FIG. 32 is a cross-sectional view schematically illustrating a cell mass-forming member according to a modified example of FIG. 3.

### Description of Embodiments

In the present specification, "to" indicating a numerical range means that numerical values described before and after the "to" are included as a lower limit value and an upper limit value.

Lower limit values and upper limit values of numerical ranges disclosed in the present specification can be freely combined to form new numerical ranges.

### Cell mass-forming member

Hereinafter, a cell mass-forming member according to an embodiment will be described with reference to a drawing as appropriate. The dimensional ratio in each drawing is for convenience of description and is different from an actual ratio. In addition, in the following drawings, the same components are denoted by the same reference numerals, and description of the same components may be omitted.

The cell mass-forming member according to an embodiment includes a substrate. The material of the substrate is not particularly limited; examples include substrates made of inorganic glass and substrates made of an organic polymer resin. Among them, the substrate is preferably a hydrophobic substrate from the viewpoint of industrial mass productivity and more preferably a resin substrate from the viewpoint of formability and production cost.

Examples of the material constituting the organic polymer resin substrate include a thermoplastic resin, a cured product of a thermosetting resin, and a cured product of a photocurable resin. Among them, the substrate is preferably a substrate containing at least one or more resins selected from the group consisting of polyethylene terephthalate) (PET), triacetyl cellulose (TAC), polycarbonate (PC), cycloolefin polymers (COPs), cycloolefin copolymers (COCs), acrylic resins (such as poly(methyl methacrylate) (PMMA)), polystyrene (PS), and dimethylpolysiloxane (PDMS). However, the material constituting the organic polymer resin substrate is not limited to these exemplifications.

Examples of the material constituting the inorganic glass substrate include quartz glasses, various alkali glasses, and various alkali-free glasses. However, the material constituting the inorganic glass substrate is not limited to these exemplifications. For shaping the fine uneven structure by a glass imprinting process, a lead borosilicate-based glass, which is a low melting point glass, or a lead-free low melting point glass is suitably used.

In addition, for example, the fine structure can also be shaped, for example, by a production method in which a UV-curable resin is applied on an alkali glass in advance and the resin coating layer is exposed in a state where a plate of the fine structure is pressed against the resin-coating layer. As in this example, a combination of an inorganic glass substrate and an organic polymer material is also effective.

The overall form of the substrate is not particularly limited. Examples include a film form, a sheet form, a plate form, and a block form.

The overall form of the substrate may be changed according to the applications of the cultured cells and/or the cell mass containing the cultured cells. For example, for producing a cell sheet having a cell mass formed on the sheet surface, the overall form of the substrate is preferably a sheet form.

On the surface of the substrate, at least one or more attachment-preventing regions and at least one or more cell attachment regions are formed. The attachment-preventing region is a region exhibiting hydrophilicity on the surface of the cell mass-forming member. On the other hand, the cell attachment region is a region exhibiting hydrophobicity on the surface of the cell mass-forming member.

This difference in the surface properties of hydrophilicity and hydrophobicity makes a difference in cell adherence between the attachment-preventing region and the cell attachment region. Thus, the cell mass-forming member according to an embodiment can selectively aggregate cells in the cell attachment region and form a collective cell mass with a three-dimensional structure in the cell attachment region.

In the cell mass-forming member according to an embodiment, a hydrophilic coating layer is formed in the attachment-preventing region. The hydrophilic coating layer is a layer of a hydrophilic material and imparts hydrophilicity to a surface of a cell culture member. The hydrophilic material is any material having a hydrophilic functional group, such as a hydroxyl group, a carbonyl group, or a carboxyl group, and is not particularly limited. Examples include biocompatible polymers, such as MPC polymers having a phospholipid-like structure.

Biocompatible polymers have low cytotoxicity and can prevent non-specific adhesion of cells and adsorption of cell adhesion factors, such as proteins. Thus, the hydrophilic material is preferably a biocompatible polymer. The type of biocompatible polymer is not particularly limited. Examples include dimethylpolysiloxane (PDMS), polyethylene glycol (PEG), oligoethylene glycol (OED), 2-methacryloyloxyethyl phosphorylcholine (MPC), poly(MPC-co-butyl methacrylate) (PMB), and poly(MPC-co-dodecyl methacrylate) (PMD).

In the cell mass-forming member according to an embodiment, the hydrophilic coating layer is provided on a part of the surface of the substrate to form the attachment-preventing region. In the cell mass-forming member in which the hydrophilic coating layer is provided, the surface of the attachment-preventing region is the surface of the hydrophilic coating layer.

The thickness of the hydrophilic coating layer provided in the attachment-preventing region is preferably from 0.005 to 0.500, more preferably from 0.005 to 0.300 µm, and even more preferably from 0.010 to 0.100 µm. With the thickness not less than the lower limit values of the above numerical ranges, the hydrophilic coating layer makes it easier for the attachment-preventing region to exhibit sufficient hydrophilicity. The configuration with the hydrophilic coating layer with the thickness not greater than the upper limit values of the above numerical ranges facilitates the reduction of the production cost.

The thickness of the hydrophilic coating layer is measured as the shortest distance between the surface of the substrate and the top surface of the hydrophilic coating layer.

In the cell mass-forming member according to an embodiment, the hydrophilic coating layer is formed in both the attachment-preventing region and the cell attachment region. In this case, the fine uneven structure region in the cell attachment region exhibits adherence to cells.

In this case, the average height of the plurality of protrusions or the average depth of the plurality of recesses in the fine uneven structure region is preferably larger than the thickness of the hydrophilic coating layer. This reduces the thickness of the coating layer of a hydrophilicity-imparting liquid on the tops of the plurality of protrusions or the basal planes of the plurality of recesses and allows cells to easily adhere.

Furthermore, reducing the thickness of the hydrophilic coating layer in the fine uneven structure region increases the adhesive force between cells and the cell mass-forming member, and increasing the thickness of the hydrophilic coating layer reduces the adhesive force between cells and the cell mass-forming member. The thickness of the hydrophilic coating layer is thus controlled in the fine uneven structure region, and this can adjust the adhesive force between cells and the cell mass-forming member accordingly. Thus, this can reproduce a state close to a cell mass in vivo corresponding to a microenvironment under set conditions.

In the cell mass-forming member according to an embodiment, in which the substrate is a resin substrate, the attachment-preventing region may be formed by introducing a hydrophilic functional group to the surface of the resin substrate. A hydrophilic functional group can be introduced to the surface of the resin substrate, for example, by irradiation treatment, such as plasma irradiation, ion irradiation, radical irradiation, or ultraviolet irradiation. The portion to which a hydrophilic functional group is introduced on the surface of the resin substrate exhibits hydrophilicity.

In the cell mass-forming member according to an embodiment, a ratio of a major axis a of an ellipse with a minimum area circumscribing the cell attachment region to a minor axis b of the ellipse is preferably from 1 to 3, more preferably from 1 to 2, and even more preferably from 1 to 1.5. The configuration with the ratio of the major axis a of the ellipse to the minor axis b in the above numerical ranges makes it easier to aggregate cells in the cell attachment region during cell culture and promotes the formation of a collective cell mass with a three-dimensional structure in which a plurality of cells aggregates. Here, for the lower limit values of the above numerical ranges, the numerical value of the ratio of the major axis a to the minor axis b can be 1. When the ratio of the major axis a to the minor axis b is 1, the ellipse with the smallest area circumscribing the cell attachment region is determined as the circumcircle of the cell attachment region.

However, in the cell mass-forming member according to another embodiment, the ratio of the major axis a of the ellipse with the minimum area circumscribing the cell attachment region to the minor axis b of the ellipse may exceed 3. The configuration with the ratio exceeding 3 in another embodiment makes it easier to obtain a cell mass in which individual cells constituting the cell mass align in the direction of the major axis.

The major axis a of the ellipse with the minimum area circumscribing the cell attachment region is preferably from 5 to 200 µm, more preferably from 10 to 100 µm, and even more preferably from 20 to 50 µm. The configuration with the major axis a of the ellipse with the minimum area circumscribing the cell attachment region not less than the lower limit values of the above numerical ranges makes it easier to promote the formation of a cell mass in the cell attachment region. The configuration with the major axis a of the ellipse with the minimum area circumscribing the cell attachment region not greater than the upper limit values of the above numerical ranges makes it easier for cells to aggregate in the cell attachment region during cell culture.

The minor axis b of the ellipse with the minimum area circumscribing the cell attachment region is preferably from 0.5 to 200 µm, more preferably from 2 to 100 µm, and even more preferably from 2 to 50 µm. The configuration with the minor axis b of the ellipse with the minimum area circumscribing the cell attachment region not less than the lower limit values of the above numerical ranges makes it easier to promote the formation of a cell mass in the cell attachment region. The configuration with the minor axis b of the ellipse with the minimum area circumscribing the cell attachment region not greater than the upper limit values of the above numerical ranges makes it easier for cells to aggregate in the cell attachment region during cell culture.

The major axis a and the minor axis b of the ellipse with the minimum area circumscribing the cell attachment region are determined as follows. A circumscribed ellipse including the cell attachment region in a plan view of the cell mass-forming member is determined, and the major axis and the minor axis of the circumscribed ellipse are measured. When the shape of the cell attachment region is irregular, a plurality of circumscribed ellipses can exist in one cell attachment region. When a plurality of circumscribed ellipses is present in one cell attachment region, the major axis and the minor axis of the circumscribed ellipse with the minimum area among these plurality of circumscribed ellipses are measured.

In a plan view of the cell mass-forming member according to an embodiment, the shapes of the attachment-preventing region and the cell attachment region are not particularly limited. The shapes of the attachment-preventing region and the cell attachment region may be, for example, a circular shape, an elliptical shape, a polygonal shape, or an irregular shape, and are not limited to these exemplifications either.

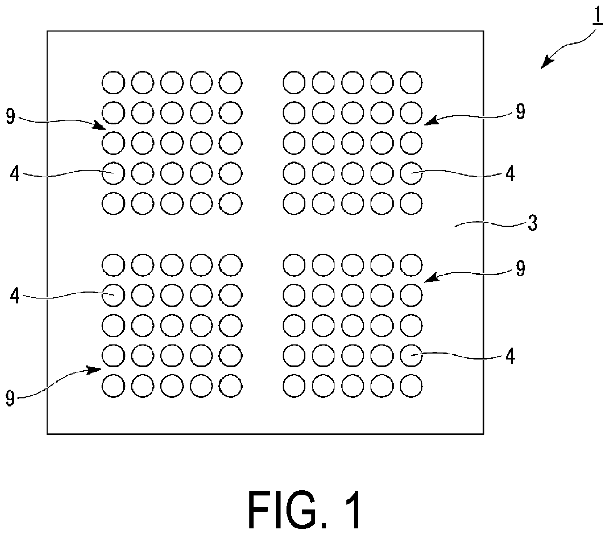

FIG. 1 is a plan view schematically illustrating a cell mass-forming member according to an embodiment. On the surface of a cell mass-forming member 1 illustrated in FIG. 1, four cell mass-forming regions 9, 9, 9, and 9 are formed. The four cell mass-forming regions 9, 9, 9, and 9 are arranged in a square lattice of 2 rows × 2 columns, and an attachment-preventing region 3 is formed between the cell mass-forming regions 9.

Each cell mass-forming region 9 includes 25 circular cell attachment regions 4 arranged in a square lattice of 5 rows × 5 columns, and the attachment preventing region 3 is also formed between the 25 cell attachment regions 4.

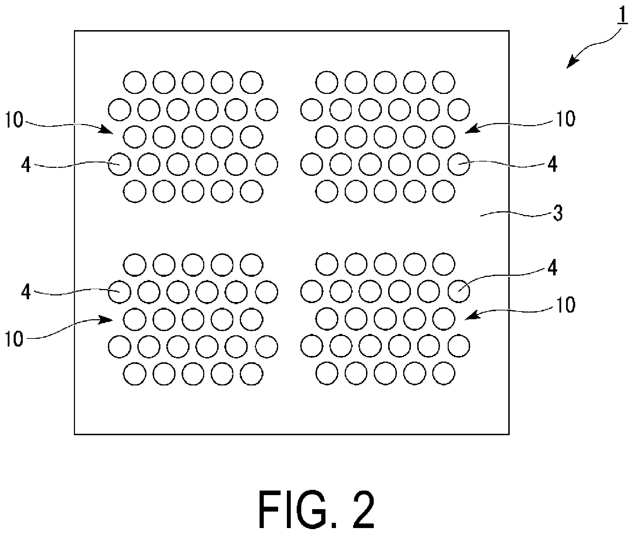

In the cell mass-forming member according to an embodiment, the arrangement mode of the cell attachment regions 4 in each cell mass-forming region is not limited to the square lattice arrangement. For example, the cell attachment regions 4 may be arranged in a triangular lattice pattern as in cell mass-forming regions 10, 10, 10, and 10 illustrated in FIG. 2.

However, in the cell mass-forming member according to an embodiment, the formation modes of the attachment-preventing region, the cell attachment region, and the cell mass-forming region are not limited to the modes illustrated in FIGS. 1 and 2. For example, although the cell mass-forming regions are regularly arranged on the surface of the cell mass-forming member in FIGS. 1 and 2, the cell mass-forming regions may be irregularly arranged.

In addition, although four cell mass-forming regions are formed in FIGS. 1 and 2, the number of the cell mass-forming regions is not particularly limited either. Similarly, the number of the cell attachment regions 4 included in each cell mass-forming member is not particularly limited either. The geometric shape of the cell attachment region 4 is not limited to the circular shape illustrated in FIGS. 1 and 2 and can be changed according to a desired form of the cell mass.

As described above, in the cell mass-forming member according to an embodiment, there can be an infinite number of modes of each of the attachment-preventing region, the cell attachment region, and the cell-forming region.

In the cell mass-forming member according to an embodiment, a fine uneven structure region including a plurality of protrusions or recesses is formed in at least a part of the cell attachment region. In the fine uneven structure region, a plurality of recesses is formed between a plurality of protrusions. Alternatively, in the fine uneven structure region, a continuous protrusion is formed between a plurality of recesses. The fine uneven structure including the plurality of protrusions and plurality of recesses can serve as a structure for supporting cells during culture. Thus, the fine uneven structure can function as a scaffold for cells like an extracellular matrix. Thus, the fine uneven structure region exhibits relatively high cell adherence in the cell attachment region and can form a three-dimensional structure.

The fine uneven structure region is to be formed in at least a part of the cell attachment region or may be formed in the entire cell attachment region. The proportion occupied by the fine uneven structure region in the cell attachment region is not particularly limited and can be changed according to a desired form and/or properties of the cell mass.

The shape of the fine uneven structure region is not particularly limited either and can be changed according to a desired form and/or properties of the cell mass. For example, when formation of a cell mass with a luminal structure is desired, the fine uneven structure region is to be formed in a ring shape. The shape of the fine uneven structure region may be changed according to a desired shape of the cell mass.

In the cell mass-forming member with the fine uneven structure region formed in a part of the cell attachment region, a difference in adherence to cells can be made also in the cell attachment region depending on the presence or absence of the fine uneven structure region. This makes it easier to further selectively aggregate cells in the fine uneven structure region in the cell attachment region, further facilitating the formation of a collective cell mass with a three-dimensional structure.

The shape of the protrusion or the recess of the fine uneven structure region is not particularly limited. The shape of the protrusion or the recess may be a conical shape, a cylindrical shape, a truncated conical shape, a pyramidal shape, or a bell shape, and is not limited to these exemplified shapes.

In the fine uneven structure region, the average height of the plurality of protrusions or the average depth of the plurality of recesses is preferably 250 nm or greater, more preferably from 300 nm to 2 µm, and even more preferably from 400 nm to 1.2 µm. With the average height of the plurality of protrusions or the average depth of the plurality of recesses not less than the lower limit values of the above numerical ranges, the protrusions and recesses in the fine uneven structure region are easily formed, increasing industrial mass productivity. In addition, when the cell mass-forming member is produced by a method (α1) described later, after a hydrophilicity-imparting liquid is applied to the entire surface of the substrate, the hydrophilicity-imparting liquid in the coating layer flows out to the attachment-preventing region, and this reduces the thickness of the coating layer of the hydrophilicity-imparting liquid on the tops of the plurality of protrusions. Thus, this improves the productivity of the cell mass-forming member and increases industrial mass productivity.

On the other hand, the plurality of protrusions with the average height or the plurality of recesses with the average depth not greater than the upper limit values of the above numerical ranges further increases cell adherence to the fine uneven structure region.

The average height of the plurality of protrusions and the average depth of the plurality of recesses are determined as follows. The cell mass-forming member is cut out perpendicularly to the surface at any desired position with a microtome or by CP processing (ion milling), or the like, and the cross section is observed with a scanning electron microscope (SEM). Twenty protrusions or recesses are randomly selected in a plurality of images in a magnification range in which from 20 to 50 protrusions or recesses are imaged, the heights of the protrusions or the depths of the recesses are measured, and their arithmetic mean value is determined as the average height of the plurality of protrusions or the average depth of the recesses.

Here, the height of each protrusion is measured as the shortest distance in the thickness direction of the substrate between the top of the protrusion and the basal plane of the fine uneven structure region, and the height of each recess is measured as the shortest distance in the thickness direction of the substrate between the top of the recess and the substrate surface.

Instead of the average height of the plurality of protrusions, the average depth of the plurality of recesses may be measured. The average depth of the plurality of recesses is determined in the same manner as the average height of the protrusions except that the depth of each recess is measured as the shortest distance between the same height position as the top of the adjacent protrusion of the recess and the basal plane of the fine uneven structure region. Details and preferred modes of the average depth of the plurality of recesses are the same as those described for the average height of the plurality of protrusions.

The average diameter of the plurality of protrusions or recesses is from 50 nm to 1.2 µm, more preferably from 100 nm to 1 µm, and even more preferably from 200 nm to 900 nm. With the average diameter of the plurality of protrusions not less than the lower limit values of the above numerical ranges, the fine uneven structure region is easily formed, increasing industrial mass productivity. The plurality of protrusions or recesses with the average diameter not greater than the upper limit values of the above numerical ranges further increases cell adherence to the fine uneven structure region.

The average diameter of the plurality of protrusions are determined as follows. The cell mass-forming member is observed in a plan view using a scanning electron microscope (SEM), 20 protrusions are randomly selected in an image in a magnification range in which from 50 to 100 of the plurality of protrusions are imaged, the major axes and the minor axes of circumscribed ellipses with the minimum area in the plan view of the 20 protrusions are measured, and the geometric mean value of the major axes and the minor axes is determined as the average diameter of the plurality of protrusions.

The average diameter of the plurality of recesses is determined in the same manner as the average diameter of the protrusions except that the major axes and the minor axes of circumscribed ellipses with the minimum area in a plan view of recesses are measured. Details and preferred modes of the average diameter of the plurality of recesses are the same as those described for the average diameter of the plurality of protrusions.

The aspect ratio of the average height of the plurality of protrusions to the average diameter of the plurality of protrusions or the aspect ratio of the average depth of the plurality of recesses to the average diameter of the plurality of recesses is preferably from 0.5 to 5.0, from 0.6 to 4.0, and even more preferably from 0.8 to 3.0.

The configuration with the aspect ratio not less than the lower limit values of the above numerical ranges further increases cell adherence to the fine uneven structure. With the aspect ratio not greater than the upper limit values of the above numerical ranges, the fine uneven structure region is easily formed, increasing industrial mass productivity.

The average pitch between the plurality of protrusions or between the plurality of recesses is preferably from 50 nm to 1 µm, more preferably from 100 nm to 900 nm, and even more preferably from 200 nm to 800 nm.

With the average pitch between the plurality of protrusions not less than the lower limit values of the above numerical ranges, the fine uneven structure region is easily formed, increasing industrial mass productivity. The configuration with the average pitch between the plurality of protrusions not greater than the upper limit values of the above numerical ranges further increases cell adherence to the fine uneven structure region.

The average pitch between the plurality of protrusions is determined as follows. The cell mass-forming member is observed in a plan view using a scanning electron microscope (SEM), 20 protrusions are randomly selected in a plurality of images in a magnification range in which from 50 to 100 of the plurality of protrusions are imaged, and the arithmetic mean value of the shortest distance between the centers of circumscribed ellipses with the minimum area in the plan view of two adjacent protrusions is determined as the average pitch of the plurality of protrusions.

The average pitch of the plurality of recesses is determined in the same manner as the average pitch of the protrusions except that the shortest distance between the centers of circumscribed ellipses with the minimum area in a plan view of two adjacent recesses is measured. Details and preferred modes of the average pitch of the plurality of recesses are the same as those described for the average pitch of the plurality of protrusions.

In an embodiment, the cell attachment region may be located at a position with a height difference relative to the attachment-preventing region in the thickness direction of the substrate. The "height difference between the attachment-preventing region and the cell attachment region" is a difference in height between the basal planes of each substrate in the attachment-preventing region and the cell attachment region. In the cell mass-forming member in which the surface of the substrate in the attachment-preventing region or the cell attachment region is a flat surface, the "basal plane of the substrate" is the flat surface itself of the substrate. In the cell mass-forming member with the fine uneven structure formed on the surface of the substrate in the attachment-preventing region or the cell attachment region, the "basal plane of the substrate" is a plane obtained by removing all the protrusions from the surface or a plane formed by filling all the recesses of the surface.

In the cell mass-forming member according to an embodiment, the positional relationship between the cell attachment region and the attachment-preventing region in the height direction (i.e., the thickness direction of the substrate) is not particularly limited. That is, the cell attachment region may be located at a higher position than the attachment-preventing region in the thickness direction of the substrate, may be located at a lower position than the attachment-preventing region, or the attachment-preventing region and the cell attachment region may be at the same height position.

Here, the height position of the cell attachment region is the height position of the top of the protrusion of the fine uneven structure region. Thus, in the cell mass-forming member with the attachment-preventing region and the cell attachment region located at the same height position, the height position of the top of the protrusion of the fine uneven structure region coincides with the height position of the surface of the attachment-preventing region in the thickness direction of the substrate.

When industrial mass productivity is required for the cell mass-forming member, the cell attachment region is to be located at a higher position than the attachment-preventing region in the thickness direction of the substrate. This is because, when the cell mass-forming member is produced by a method (α1) described later, after a hydrophilicity-imparting liquid is applied to the entire surface of the substrate, the hydrophilicity-imparting liquid easily flows down from the cell attachment region to the attachment-preventing region, facilitating the production.

In the cell mass-forming member according to an embodiment, the height difference between the cell attachment region and the surface of the substrate is preferably from 0 to 3 µm, more preferably from 100 nm to 2 µm, and even more preferably from 300 nm to 1 µm. With the height difference greater than 0 µm and the cell attachment region located at a higher position than the attachment-preventing region, when the cell mass-forming member is produced by a method (α1) described later, after a hydrophilicity-imparting liquid is applied to the entire surface of the substrate, the hydrophilicity-imparting liquid easily flows down from the cell attachment region to the attachment-preventing region. Thus, this facilitates the production of the cell mass-forming member, further increasing industrial mass productivity. The configuration with the height difference not greater than the upper limit values of the above numerical ranges is less likely to inhibit the movement of cells to the cell attachment region.

In the cell mass-forming member according to an embodiment, a hydrophilic layer may be formed in recesses between the plurality of protrusions of the fine uneven structure region of the cell attachment region or in bottoms of recesses between the plurality of protrusions. The hydrophilic layer is a layer of hydrophilic material. Details and preferred modes of the hydrophilic material are the same as those described for the hydrophilic coating layer. The hydrophilic material in the hydrophilic layer may be the same material as or a different material from the hydrophilic material of the hydrophilic coating layer. In view of industrial mass productivity, the hydrophilic material in the hydrophilic layer is preferably the same material as the hydrophilic material of the hydrophilic coating layer.

In the cell mass-forming member according to an embodiment, a hydrophilic layer may be formed in recesses between the plurality of protrusions of the fine uneven structure region or in bottoms of recesses between the plurality of protrusions. In this case, the fine uneven structure region in the cell attachment region exhibits adherence to cells.

In the cell mass-forming member according to an embodiment, a layer of an adhesion factor may be provided on the surface of the cell attachment region to enhance cell adherence. Examples of the adhesion factor include extracellular matrices, such as laminin, collagen, gelatin, fibronectin, polylysine (PDL, PLL), and hyaluronic acid; polymers; and gels.

### Effects

In the cell mass-forming member according to an embodiment described above, at least one or more attachment-preventing regions and at least one or more cell attachment regions are formed on the surface of the substrate, and the fine uneven structure region is formed in at least a part of the cell attachment region. Thus, cells relatively easily adhere to the cell attachment region compared with the attachment preventing region, and cells more easily adhere to the fine uneven structure region in the cell attachment region. The sufficient differences in adherence to cells thus made between the attachment-preventing region, the cell attachment region, and the fine uneven structure region increases the tendency of cells to move from the attachment-preventing region to the fine uneven structure region in the cell attachment region during cell culture.

Thus, the cell mass-forming member according to an embodiment facilitates the formation of a cell mass.

### Production method

The method for producing the cell mass-forming member according to an embodiment is not particularly limited. For example, the cell mass-forming member can be produced by a method (α) below.

Method (α): dividing a surface of a substrate into at least one or more attachment-preventing regions and at least one or more cell attachment regions, forming a fine uneven structure region in at least a part of the cell attachment region, and subjecting the attachment-preventing region to hydrophilization treatment.

In the method (α), the division between the attachment-preventing region and the cell attachment region can be changed according to the type of cell and the form and properties of a desired cell mass, and is not particularly limited.

For example, a single particle film etching mask made of colloidal silica described in JP 2009-034630 A may be used to form the fine uneven structure region. The fine uneven structure region can be formed on the substrate by vapor phase etching using the single particle film etching mask. In addition, various methods for forming the fine uneven structure region can be used, including colloidal lithography, anodic oxidation, or interference exposure.

The hydrophilization treatment may be any treatment for increasing hydrophilicity and is not particularly limited. For example, a hydrophilicity-imparting liquid containing at least a hydrophilic material may be applied to the surface of the substrate. Thereafter, the coating layer of the hydrophilicity-imparting liquid may be solidified to form a hydrophilic coating layer on a part of the surface of the substrate and form at least one or more attachment-preventing regions and at least one or more cell attachment regions.

When a hydrophobic substrate is used, a part of the surface of the substrate may be subjected to hydrophilization treatment to form at least one or more attachment-preventing regions and at least one or more cell attachment regions on the surface of the substrate.

When a resin substrate is used, the surface of the resin substrate may be subjected to an irradiation treatment, such as plasma irradiation, ion irradiation, radical irradiation, or ultraviolet irradiation, as the hydrophilization treatment. The irradiation treatment resulted in breaking chemical bonds of molecules on the surface of the substrate, forming a hydrophilic functional group corresponding to the type of resin, and improving the hydrophilicity compared with that before the treatment. Among the irradiation treatments, plasma irradiation is preferred in terms of less physical damage to the substrate surface.

To facilitate detachment and recovery of cultured cells, a stimulus-responsive material may be applied to the surface of the cell mass-forming member. The stimulus-responsive material is preferably a temperature-responsive polymer with hydrophilicity that changes according to temperature change and preferably poly(N-isopropylacrylamide) (PIPAAm).

When the stimulus-responsive material is applied to the substrate, the fine uneven structure region may be formed after the stimulus-responsive material is applied to the substrate, or the stimulus-responsive material may be applied to the substrate after the fine uneven structure region is formed. However, when the stimulus-responsive material is applied to the substrate after the fine uneven structure region is formed, the stimulus-responsive material is preferably applied to the substrate before the hydrophilization treatment.

Examples of the method (α) include a method (α1) below as a preferred example from the viewpoint of industrial mass productivity.

Method (α1): dividing a surface of a substrate into an attachment-preventing region and a cell attachment region, a fine uneven structure region being formed, and then a hydrophilicity-imparting liquid is applied to the entire surface of the substrate.

In the method (α1), when the attachment-preventing region and the cell attachment region are divided from each other, the cell attachment region is to be formed at a higher position than the attachment-preventing region in the thickness direction of the substrate. This is because after the hydrophilicity-imparting liquid is applied to the entire surface of the substrate, the hydrophilicity-imparting liquid easily flows down from the cell attachment region to the attachment-preventing region, facilitating the production.

In the cell mass-forming member with the cell attachment region formed at a higher position than the attachment-preventing region in the thickness direction of the substrate, a height difference in the thickness direction may be provided on the surface of the substrate. This height difference is to be provided in the substrate by a typical photolithography method.

### Embodiment examples

Hereinafter, the cell mass-forming member according to an embodiment will be described in detail by exemplifying embodiments, but the present invention is not limited to these embodiment examples.

### Embodiment Example 1

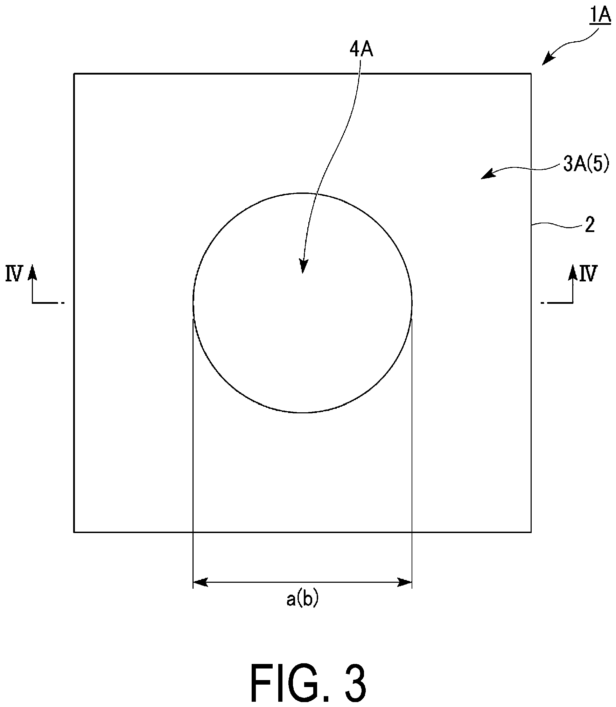

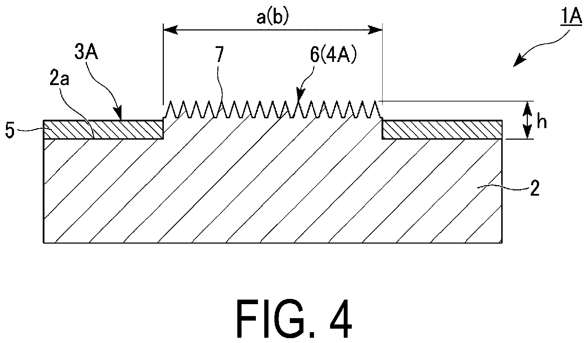

FIG. 3 is an enlarged plan view schematically illustrating an example of a cell mass-forming member. FIG. 4 is a cross-sectional view taken along line IV-IV of FIG. 3.

A cell mass-forming member 1A illustrated in FIGS. 3 and 4 has a substrate 2. A circular and spot-like cell attachment region 4A and an attachment-preventing region 3A are formed on a surface 2a of the substrate 2. Thus, this makes a difference in cell adherence in each region. Thus, the cell mass-forming member 1A can selectively aggregate cells in the cell attachment region 4A and form a cell mass in the circular and spot-like cell attachment region.

In the cell mass-forming member 1A, the attachment-preventing region 3A is formed outside the circular and spot-like cell attachment region 4A. In addition, the attachment-preventing region 3A is covered with a hydrophilic coating layer 5 provided on the surface 2a of the substrate 2. The attachment-preventing region 3A exhibits hydrophilicity due to this hydrophilic coating layer 5. The thickness of the hydrophilic coating layer 5 in the attachment-preventing region 3A is preferably from 0.005 to 0.500 µm, more preferably from 0.005 to 0.300 µm, and even more preferably from 0.010 to 0.100 µm.

The spot-like cell attachment region 4A has a circular shape in a plan view. In this case, an ellipse of the minimum area circumscribing the cell attachment region 4A coincides with a circumferential circle dividing the cell attachment region 4A and the attachment-preventing region 3A from each other. Thus, in the cell mass-forming member 1A, the major axis a of the ellipse with the minimum area circumscribing the cell attachment region 4A coincides with the minor axis b of the ellipse and is equal to the diameter of the circumferential circle dividing the cell attachment region 4A and the attachment-preventing region 3A from each other.

In the cell mass-forming member 1A, the entire cell attachment region 4A is a fine uneven structure region 6. The cell attachment region 4A exhibits hydrophobicity due to the fine uneven structure region 6 formed on the surface 2a of the substrate 2. The fine uneven structure region 6 includes a plurality of conical protrusions 7.

In the cell mass-forming member 1A, the cell attachment region 4A is located at a higher position than the attachment-preventing region 3A in the thickness direction of the substrate 2. Thus, in producing the cell mass-forming member by the method (α1), when the hydrophilicity-imparting liquid is applied to the entire surface 2a of the substrate 2, the hydrophilicity-imparting liquid easily flows down from the cell attachment region 4A to the attachment-preventing region 3A, facilitating the production.

A height difference h between the cell attachment region 4A and the surface 2a of the substrate 2 is preferably from 0 to 3 µm, more preferably from 100 nm to 2 µm, and even more preferably from 300 nm to 1 µm. With the height difference h not less than the lower limit values of the above numerical ranges, the cell mass-forming member is easily produced, increasing industrial mass productivity. The configuration with the height difference h not greater than the upper limit values of the above numerical ranges is less likely to inhibit the movement of cells to the cell attachment region.

In the cell mass-forming member 1A, a sufficient difference in adherence to cells can be obtained between the attachment-preventing region 3A and the fine uneven structure region 6. Thus, this increases the tendency of cells to move from the attachment-preventing region 3A to the fine uneven structure region in the cell attachment region 4A during cell culture.

The configuration with a sufficiently small ratio of the major axis a to the minor axis b of the ellipse with the minimum area circumscribing the spot-like cell attachment region 4A makes it easier to aggregate cells in the spot-like cell attachment region 4A during cell culture, promoting the formation of a collective cell mass with a three-dimensional structure in which a plurality of cells aggregates.

Thus, the cell mass-forming member 1A facilitates the formation of a cell mass.

### Embodiment Example 2

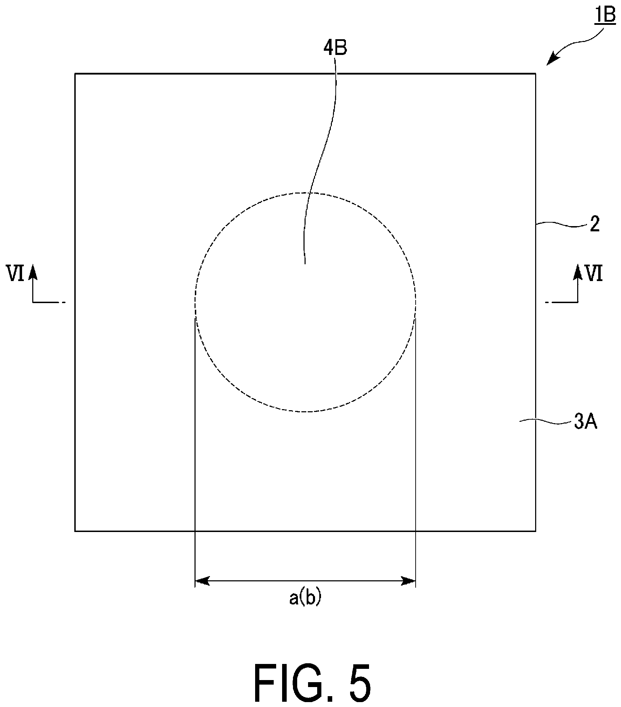

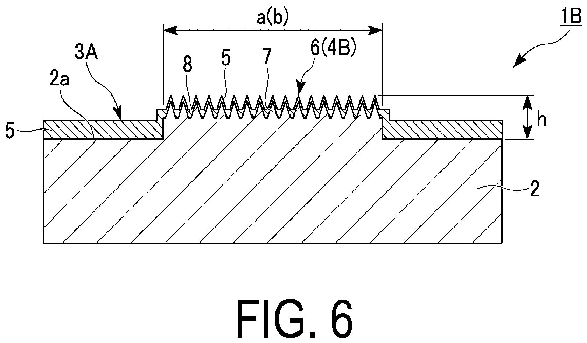

FIG. 5 is an enlarged plan view schematically illustrating an example of a cell mass-forming member. FIG. 6 is a cross-sectional view taken along line VI-VI of FIG. 5.

A cell-mass forming member 1B illustrated in FIGS. 5 and 6 is different from the cell-mass forming member 1A in the following three points and has a configuration common to the cell-mass forming member 1A in other points.
· The hydrophilic coating layer 5 is also formed on the surfaces of the plurality of protrusions 7 of the fine uneven structure region 6 in a cell attachment region 4B.
· A hydrophilic layer 8 is formed in bottoms of recesses between the plurality of protrusions 7 of the fine uneven structure region 6B.
· The surface of the hydrophilic coating layer 5 formed in the fine uneven structure region 6 of the cell attachment region 4B conforms to the fine uneven structure region 6.

In the cell mass-forming member 1B, the average height of the plurality of protrusions 7 or the average depth of the plurality of recesses is larger than the thickness of the hydrophilic coating layer in the fine uneven structure region 6.

The cell mass-forming member 1B can be suitably produced by the method (α1). It can also be said that the hydrophilic coating layer 5 in the fine uneven structure region 6 of the cell attachment region 4B is formed by solidification of a remaining portion of a hydrophilicity-imparting liquid applied to the entire surface 2a of the substrate 2, the remaining portion remaining on the surfaces of the plurality of protrusions or the plurality of recesses.

The thickness of the hydrophilic coating layer 5 in the fine uneven structure region 6 of the cell attachment region 4B is preferably from 0.005 to 0.500 µm, more preferably from 0.005 to 0.300 µm, and even more preferably from 0.010 to 0.100 µm.

The hydrophilic layer 8 is a layer of a hydrophilic material. Details and preferred modes of the hydrophilic material are the same as those described for the hydrophilic coating layer. The hydrophilic material in the hydrophilic layer 8 may be the same as or a different material from the hydrophilic material of the hydrophilic coating layer 5. However, in view of industrial mass productivity, both the layers are preferably made of the same hydrophilic material.

Also in the cell mass-forming member 1B, the same effects as those of the cell mass-forming member 1A are obtained. In addition, for the cell mass-forming member 1B, the attachment-preventing region 3A and the cell attachment region 4B can be formed by painting all over the entire surface 2a of the substrate 2 with a hydrophilicity-imparting liquid in the production process. Thus, the cell mass-forming member 1B also has excellent industrial mass productivity.

### Embodiment Example 3

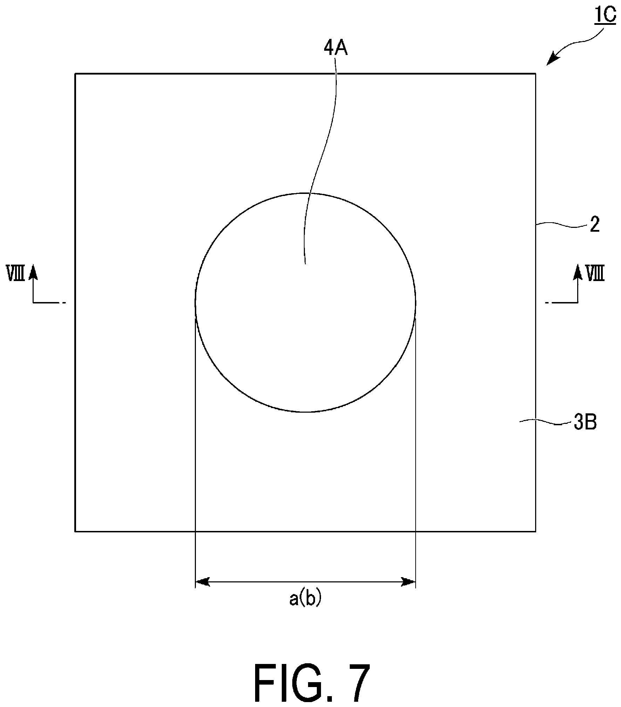

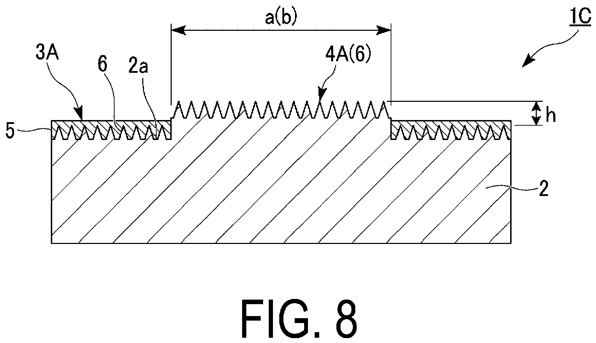

FIG. 7 is an enlarged plan view schematically illustrating an example of a cell mass-forming member. FIG. 8 is a cross-sectional view taken along line VIII-VIII of FIG. 7. A cell-mass forming member 1C illustrated in FIGS. 7 and 8 is different from the cell-mass forming member 1A in that the fine uneven structure region 6 is also formed in an attachment-preventing region 3B, and has a configuration common to the cell-mass forming member 1A in other points.

In the cell mass-forming member 1C, the "height difference between the cell attachment region and the surface of the substrate" is the difference in height between the basal planes of the plurality of protrusions of the fine uneven structure region 6 in the cell attachment region 4A and the basal planes of the plurality of protrusions of the fine uneven structure region 6 in the attachment-preventing region 3B.

The details and preferred modes of the fine uneven structure region 6 in the attachment-preventing region 3B are the same as those of the fine uneven structure region 6 described for the cell mass-forming member 1A except that the average height of the plurality of protrusions 7 is not greater than the thickness of the hydrophilic coating layer 5. The average height of the plurality of protrusions 7 in the attachment-preventing region 3B is preferably 80% or less and desirably 50% or less relative to the hydrophilic coating layer 5.

Properties, such as the average height, the average diameter, and the average pitch, of the protrusions 7 of the fine uneven structure region 6 in the attachment-preventing region 3B may be the same as or different from the properties of the fine uneven structure region 6 in the cell attachment region 4A.

Also in the cell mass-forming member 1C, the same effects as those of the cell mass-forming member 1A are obtained.

### Embodiment Example 4

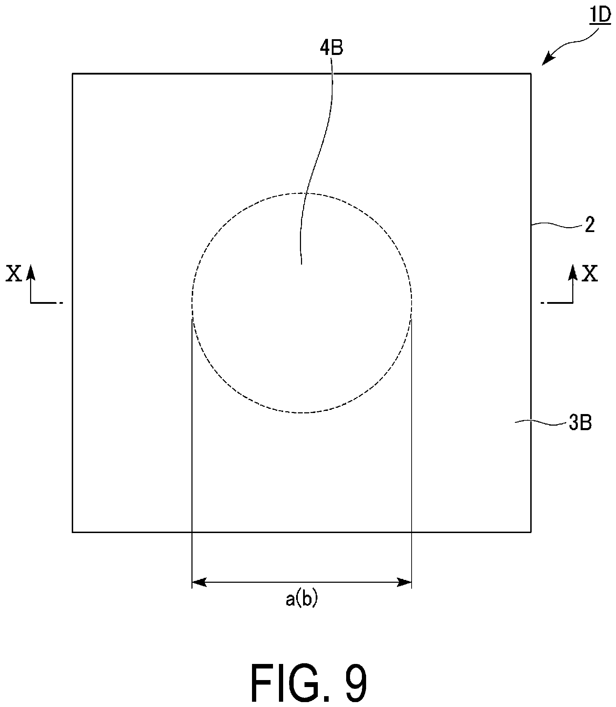

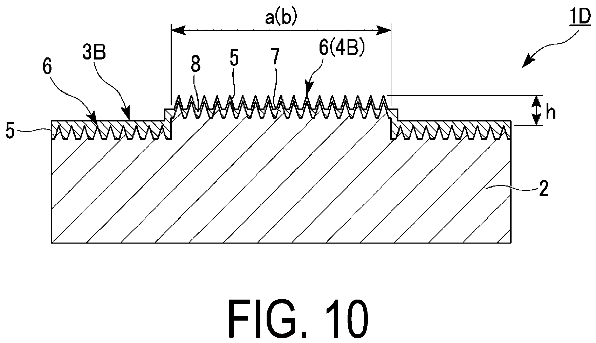

FIG. 9 is an enlarged plan view schematically illustrating an example of a cell mass-forming member. FIG. 10 is a cross-sectional view taken along line X-X of FIG. 9. A cell-mass forming member 1D illustrated in FIGS. 9 and 10 is different from the cell-mass forming member 1A in the following three points and has a configuration common to the cell-mass forming member 1A in other points.
· The fine uneven structure region 6 is formed in the attachment-preventing region 3B.
· The hydrophilic coating layer 5 is also formed on the surfaces of the plurality of protrusions 7 of the fine uneven structure region 6 of the cell attachment region 4B.
· The surface of the hydrophilic coating layer 5 formed in the fine uneven structure region 6 of the cell attachment region 4B conforms to the fine uneven structure region 6.

In the cell mass-forming member 1D, the average height of the plurality of protrusions 7 or the average depth of the plurality of recesses is larger than the thickness of the hydrophilic coating layer in the fine uneven structure region 6.

The size relationship between the average height of the plurality of protrusions 7 or the average depth of the plurality of recesses and the thickness of the hydrophilic coating layer in the fine uneven structure region 6 can be confirmed by observing the surface of the fine uneven structure region 6 with an electron microscope to confirm that the fine uneven structure appears on the surface. Alternatively, the size relationship between the average height of the plurality of protrusions 7 or the average depth of the plurality of recesses and the thickness of the hydrophilic coating layer can be confirmed by preparing a cross-sectional sample of the cell mass-forming member by embedding the fine uneven structure region 6 with an embedding resin for cross-sectional observation, and observing the cross section with an electron microscope.

Also in the cell mass-forming member 1D, the same effects as those of the cell mass-forming member 1A are obtained. In addition, the cell mass-forming member 1D also has excellent industrial mass productivity like the cell mass-forming member 1B.

### Embodiment Example 5

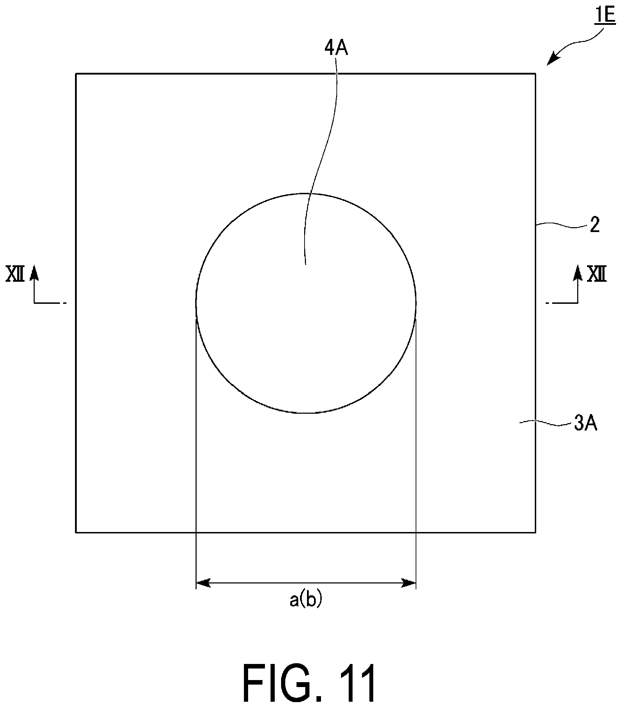

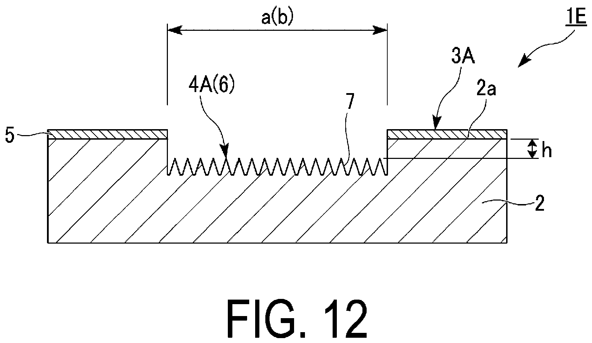

FIG. 11 is an enlarged plan view schematically illustrating an example of a cell mass-forming member. FIG. 12 is a cross-sectional view taken along line XII-XII of FIG. 11.

A cell-mass forming member 1E illustrated in FIGS. 11 and 12 is different from the cell-mass forming member 1A in that the cell attachment region 4A is located at a lower position than the surface 2a of the substrate 2 in the thickness direction of the substrate 2, and has a configuration common to the cell mass-forming member 1A in other points.

Even a configuration like the cell mass-forming member 1E, where the cell attachment region 4A is depressed and located at a lower position than the attachment-preventing region 3A, has a strong tendency of cells to move from the attachment-preventing region 3A to the cell attachment region 4A during cell culture, and the same effects as those of the cell mass-forming member 1A are obtained.

### Embodiment Example 6

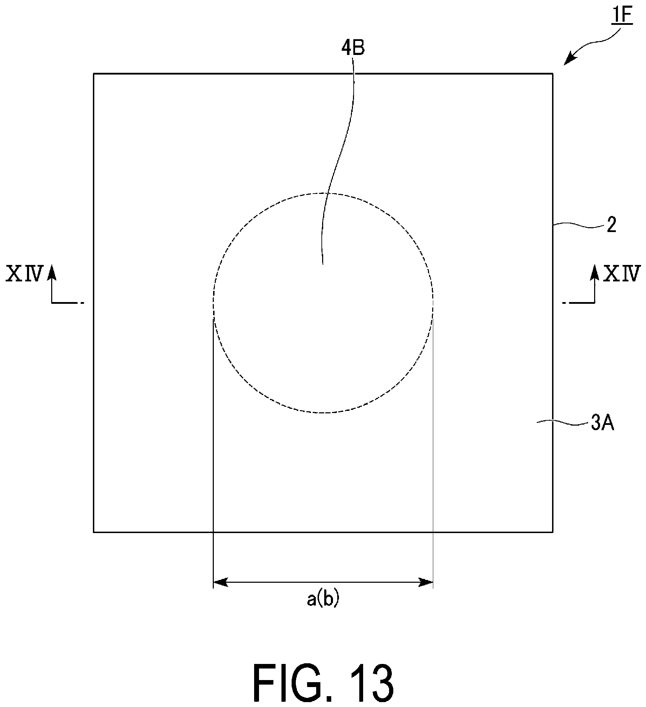

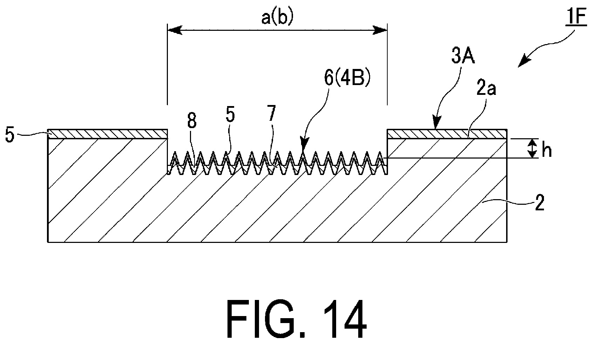

FIG. 13 is an enlarged plan view schematically illustrating an example of a cell mass-forming member. FIG. 14 is a cross-sectional view taken along line XIV-XIV of FIG. 13.

A cell-mass forming member 1F illustrated in FIGS. 13 and 14 is different from the cell-mass forming member 1B in that the cell attachment region 4B is located at a lower position than the surface 2a of the substrate 2 in the thickness direction of the substrate 2, and has a configuration common to the cell mass-forming member 1B in other points.

Also in the cell mass-forming member 1F, the same effects as those of the cell mass-forming member 1B are obtained.

### Embodiment Example 7

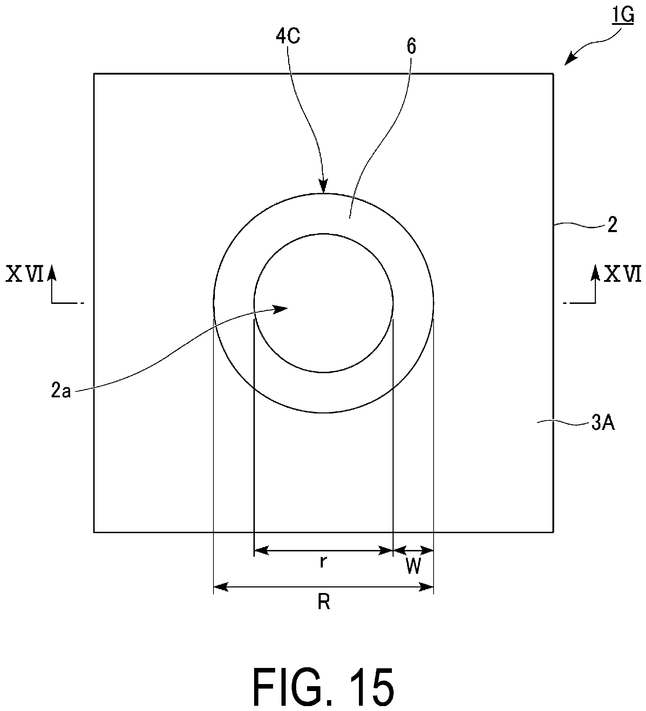

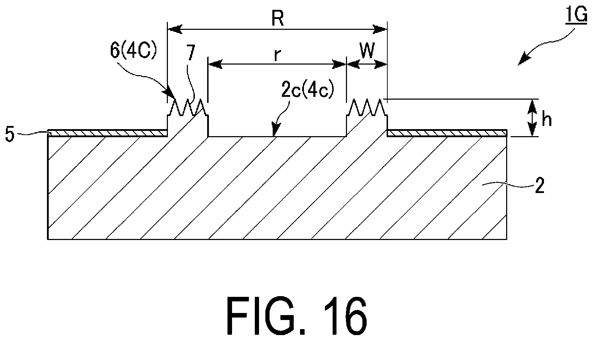

FIG. 15 is an enlarged plan view schematically illustrating an example of a cell mass-forming member. FIG. 16 is a cross-sectional view taken along line XVI-XVI of FIG. 15.

In a cell mass-forming member 1G illustrated in FIGS. 15 and 16, the attachment-preventing region 3A and a cell attachment region 4C are formed in the surface 2a of the substrate 2. In a plan view of the cell mass-forming member 1G, the fine uneven structure region 6 is annularly formed in the cell attachment region 4C.

The outer periphery of the annular fine uneven structure region 6 coincides with the outer edge of the cell attachment region 4C. In addition, in the region inside the inner periphery of the annular fine uneven structure region 6, the surface 2a of the substrate 2 is exposed to be a part of the cell attachment region 4C. In the cell mass-forming member 1G, the surface of the cell attachment region 4C is configured to include the surface of the annular fine uneven structure region 6 and the surface 2a of the substrate 2.

The diameter R of the outer diameter circle of the annular fine uneven structure region 6 is preferably from 10 to 100 µm, more preferably from 10 to 60 µm, and even more preferably from 20 to 45 µm. With the diameter R of the outer diameter circle not less than the lower limit values of the above numerical ranges, the annular fine uneven structure region 6 is easily formed, increasing industrial mass productivity. The configuration with the diameter R of the outer diameter circle not greater than the upper limit values of the above numerical ranges makes it easier to selectively aggregate cells in the cell attachment region 4C and form a cell mass.

The diameter r of the inner diameter circle of the annular fine uneven structure region 6 is preferably 500 nm or greater, more preferably 1 µm or greater, and even more preferably 10 µm or greater. With the diameter r of the inner diameter circle not less than the lower limit values of the above numerical ranges, the annular fine uneven structure region 6 is easily formed, increasing industrial mass productivity and making it easier to promote the formation of a cell mass with a three-dimensional structure in the annular fine uneven structure region 6.

The width of the annular fine uneven structure region 6, that is, the difference between the outer diameter R and the diameter r of the inner diameter circle is preferably from 200 nm to 30 µm, more preferably from 1 to 20 µm, and even more preferably from 3 to 15 µm. With the width W not less than the lower limit values of the above numerical ranges, the annular fine uneven structure region 6 is easily formed, increasing industrial mass productivity. The configuration with the width W not greater than the upper limit values of the above numerical ranges makes it easier to promote the formation of a collective cell mass with a three-dimensional structure in the annular fine uneven structure region 6.

In the cell attachment region 4C, the surfaces of the protrusions 7 of the annular fine uneven structure region 6 tend to exhibit relatively high adherence to cells. In the cell mass-forming member 1G, the difference in adherence to cells is thus easily obtained not only between the attachment-preventing region 3A and the cell attachment region 4C but also by the presence or absence of the fine uneven structure region 6 in the cell attachment region 4C.

Thus, not so much hydrophilicity need be imparted to the attachment-preventing region 3A in the cell mass-forming member 1G as to the attachment-preventing region 3A in the cell mass-forming member 1A. As a result, the thickness of the hydrophilic coating layer 5 of the attachment-preventing region 3A in the cell mass-forming member 1G may be smaller than that of the hydrophilic coating layer of the attachment-preventing region 3A in the cell mass-forming member 1A.

From this point of view, the thicknesses of the hydrophilic coating layer 5 in the attachment-preventing region 3A in the cell mass-forming member 1G is preferably from 0.005 to 0.500 µm, more preferably from 0.005 to 0.300 µm, and even more preferably from 0.010 to 0.100 µm. With the thickness not less than the lower limit values of the above numerical ranges, the hydrophilic coating layer 5 of the attachment-preventing region 3A in the cell mass-forming member 1G easily imparts sufficient hydrophilicity to the attachment-preventing region 3A in the cell mass-forming member 1G. The configuration with the hydrophilic coating layer 5 of the attachment-preventing region 3A in the cell mass-forming member 1G with the thickness not greater than the upper limit values of the above numerical ranges facilitates the reduction of the production cost.

Also in the cell mass-forming member 1G, the same effects as those of the cell mass-forming member 1A are obtained. In addition, in the cell mass-forming member 1G, cells are likely to aggregate on the surfaces of the protrusions 7 of the fine uneven structure region 6 in the cell attachment region 4C during cell culture. As a result, a cell mass is easily formed annularly on the surface of the annular fine uneven structure region 6, facilitating the formation of a cell mass with a luminal structure.

Although further illustration is omitted, in another embodiment example, the annular fine uneven structure region 6 may be depressed and located at a lower position than the attachment-preventing region 3A. Also in this case, the same effects as those of the cell mass-forming member 1G are obtained.

### Embodiment Example 8

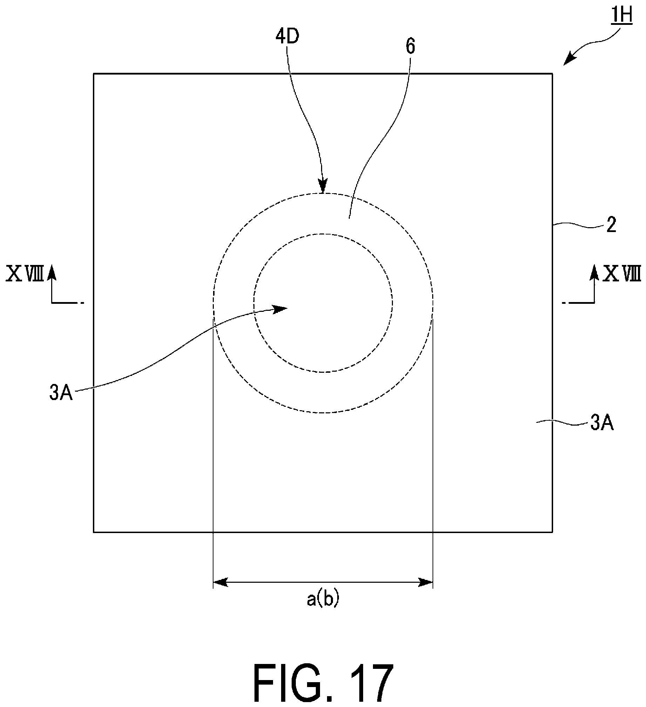

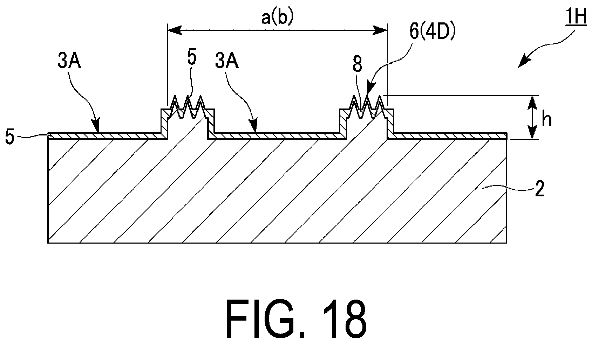

FIG. 17 is an enlarged plan view schematically illustrating an example of a cell mass-forming member. FIG. 18 is a cross-sectional view taken along line XVIII-XVIII of FIG. 17. A cell-mass forming member 1H illustrated in FIGS. 17 and 18 is different from the cell-mass forming member 1G in the following two points and has a configuration common to the cell-mass forming member 1G in other points.
· The hydrophilic coating layer 5 is also formed in recesses between the plurality of protrusions 7 of a ring-shaped fine uneven structure region 6.
· The attachment-preventing region 3A is also formed in a region inside the ring-shaped fine uneven structure region 6.

Also in the cell mass-forming member 1H, the same effects as those of the cell mass-forming member 1G are obtained. In addition, the cell mass-forming member 1H can be suitably produced by the method (α1) in the same manner as the cell mass-forming member 1B to have the hydrophilic coating layer 5 and thus also has excellent industrial mass productivity.

Although further illustration is omitted, in another embodiment example, the ring-shaped fine uneven structure region 6 may be depressed and located at a lower position than the attachment-preventing region 3A. Also in this case, the same effects as those of the cell mass-forming member 1H are obtained.

### Embodiment Example 9

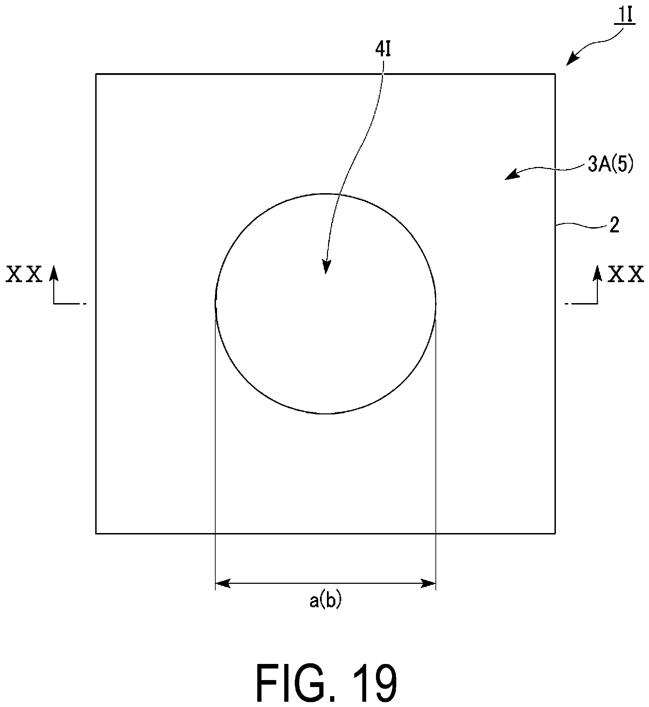

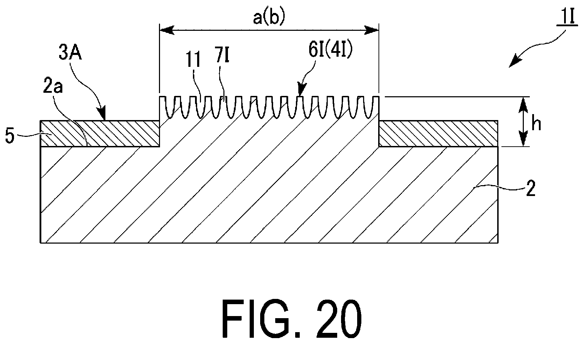

FIG. 19 is an enlarged plan view schematically illustrating an example of a cell mass-forming member. FIG. 20 is a cross-sectional view taken along line XX-XX of FIG. 19. A cell-mass forming member 1I illustrated in FIGS. 19 and 20 is different from the cell-mass forming member 1A in that a recessed fine uneven structure region 6I including a plurality of recesses 11 is formed, and has a configuration common to the cell-mass forming member 1A in other points.

Also in the cell mass-forming member 1I, the same effects as those of the cell mass-forming member 1A are obtained.

### Embodiment Example 10

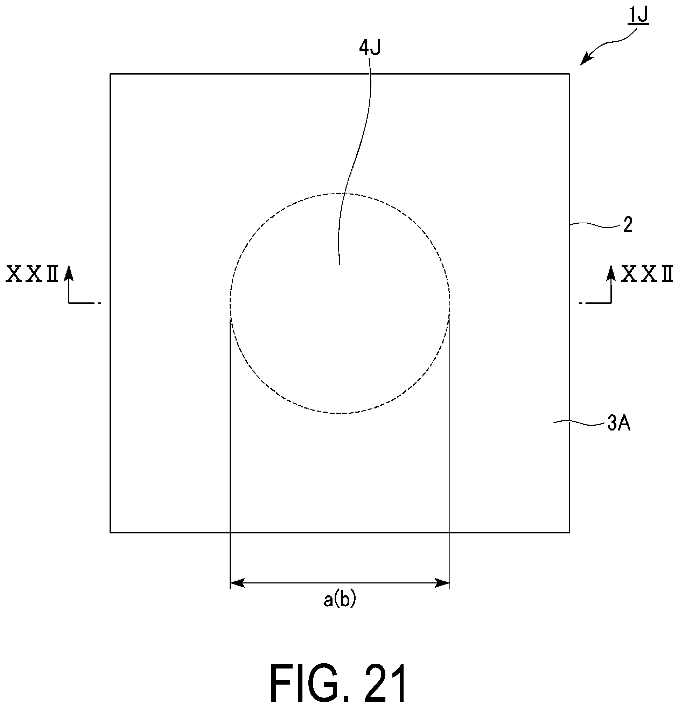

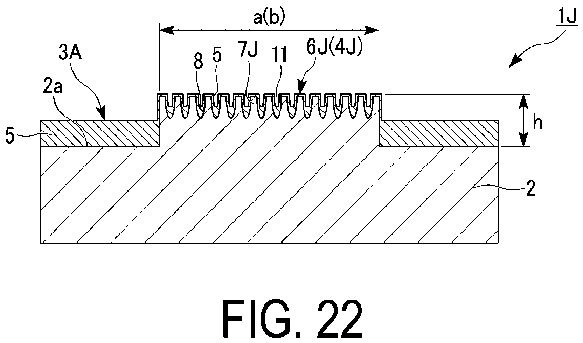

FIG. 21 is an enlarged plan view schematically illustrating an example of a cell mass-forming member. FIG. 22 is a cross-sectional view taken along line XXII-XXII in FIG. 21. A cell-mass forming member 1J illustrated in FIGS. 21 and 22 is different from the cell-mass forming member 1B in the following four points and has a configuration common to the cell-mass forming member 1B in other points.
· A recessed fine uneven structure region 6J is formed.
· The hydrophilic layers 8 are formed in bottoms of the plurality of recesses 11 of the fine uneven structure region 6.
· The surface of the hydrophilic coating layer 5 formed in the fine uneven structure region 6 of the cell attachment region 4B conforms to the fine uneven structure region 6.
· In the cell mass-forming member 1J, the average height of a plurality of protrusions 7J or the average depth of the plurality of recesses is larger than the thickness of the hydrophilic coating layer in the fine uneven structure region 6.

Also in the cell mass-forming member 1J, the same effects as those of the cell mass-forming member 1B are obtained.

### Embodiment Example 11

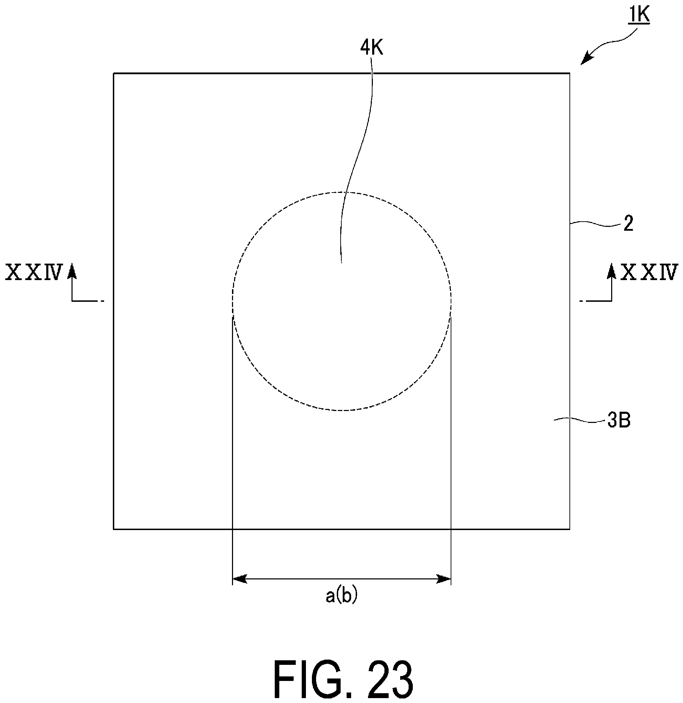

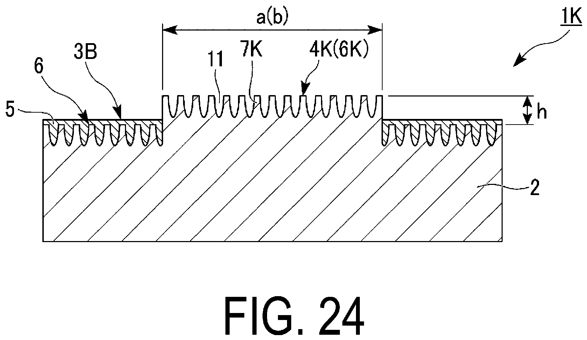

FIG. 23 is an enlarged plan view schematically illustrating an example of a cell mass-forming member. FIG. 24 is a cross-sectional view taken along line XXIV-XXIV of FIG. 23. A cell-mass forming member 1K illustrated in FIGS. 23 and 24 is different from the cell-mass forming member 1C in that a recessed fine uneven structure region 6K including the plurality of recesses 11 is formed, and has a configuration common to the cell-mass forming member 1C in other points.

Also in the cell mass-forming member 1K, the same effects as those of the cell mass-forming member 1C are obtained.

### Embodiment Example 12

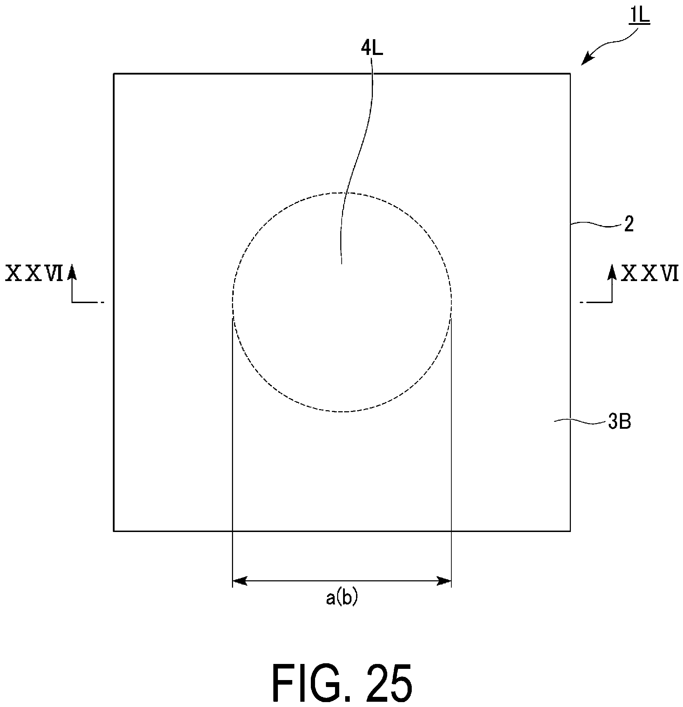

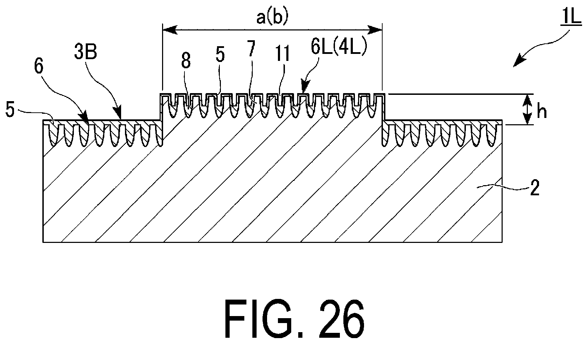

FIG. 25 is an enlarged plan view schematically illustrating an example of a cell mass-forming member. FIG. 26 is a cross-sectional view taken along line XXVI-XXVI of FIG. 25. A cell-mass forming member 1L illustrated in FIGS. 25 and 26 is different from the cell-mass forming member 1D in the following two points and has a configuration common to the cell-mass forming member 1D in other points.
· A recessed fine uneven structure region 6L is formed.
· The hydrophilic layers 8 are formed in bottoms of the plurality of recesses 11 of the fine uneven structure region 6.

Also in the cell mass-forming member 1L, the same effects as those of the cell mass-forming member 1D are obtained.

### Embodiment Example 13

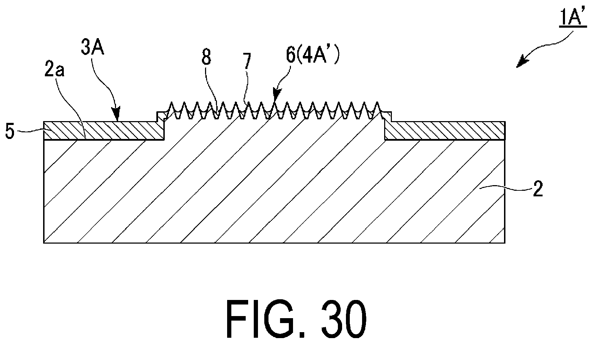

FIG. 30 is a cross-sectional view corresponding to the cross-section taken along line IV-IV in a modified example (1A') of the cell mass-forming member 1A illustrated in FIG. 3. In this example, the tips of the plurality of protrusions 7 are exposed from the hydrophilic layer 8 in the fine uneven structure region 6 of a cell attachment region 4A'. The cell mass-forming member according to this example further increases the cell attachment properties in the cell attachment region 4A'.

### Embodiment Example 14

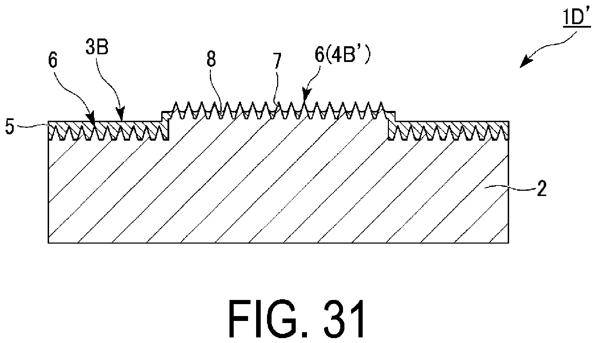

FIG. 31 is a cross-sectional view corresponding to the cross-section taken along line X-X in a modified example (1D') of the cell mass-forming member 1D illustrated in FIG. 9. In this example, the tips of the plurality of protrusions are exposed from the hydrophilic layer 8 in the fine uneven structure region 6 of a cell attachment region 4B'. The cell mass-forming member according to this example further increases the cell attachment properties in the cell attachment region 4B'.

### Embodiment Example 15

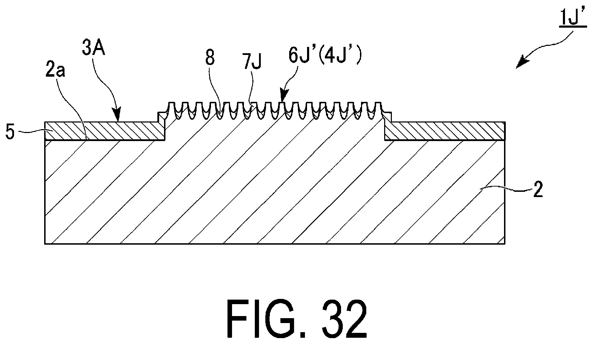

FIG. 32 is a cross-sectional view corresponding to the cross-section taken along line XXII-XXII in a modified example (1J') of the cell mass-forming member 1J illustrated in FIG. 21. In this example, the tips of the plurality of protrusions 7J are exposed from the hydrophilic layer 8 in a cell attachment region 4J'. This example further increases the cell attachment properties in the cell attachment region 4J'.

### Culture vessel

A culture vessel according to an embodiment includes the cell mass-forming member according to an embodiment and thus can easily form a cell mass and has excellent industrial mass productivity.

Examples of the culture vessel include vessels provided with a cell mass-forming member on an inner wall surface of a vessel main body for containing a culture solution containing cells. The inner wall surface of the vessel main body is any surface that can come into contact with a culture solution and is not particularly limited and may be a bottom surface of the vessel main body or an inner side surface of the vessel main body. The cell mass-forming member may be provided on the inner wall surface of the vessel main body with an adhesive.

The shape and size of the culture vessel are not particularly limited. Examples of the culture vessel include Petri dishes, culture plates with one or a plurality of wells (holes), culture flasks; and slide glass chambers.

The number of wells of the culture plate is not particularly limited and is set according to the use of cultured cells, an analyzer to be used, and/or the like. The shape of the well in a plan view is not particularly limited. Examples of the shape include a perfect circle, an ellipse, a triangle, a square, a rectangle, and a pentagon. The shape of the bottom surface of the well is not particularly limited either, and examples include a flat bottom, a round bottom, and an unevenness.

The material of the culture vessel is not particularly limited, and examples include polymer materials, metal materials, and inorganic materials. Examples of the polymer resin include polystyrene, polyethylene, polypropylene, polycarbonate, polyester, polyisoprene, a cycloolefin polymers, polyimide, polyamide, poly(amide-imide), (meth)acrylic resins, epoxy resins, and silicone.

Examples of the metal material include stainless steel, copper, iron, nickel, aluminum, titanium, gold, silver, and platinum.

Examples of the inorganic material include silicon oxide (glass), aluminum oxide, titanium oxide, zirconium oxide, iron oxide, and silicon nitride.

### Method for producing cultured cells

In a method for producing cultured cells according to an embodiment, cells are cultured using the cell mass-forming member according to an embodiment, and then the cultured cells are detached from the cell mass-forming member. In the method for producing cultured cells, cells are cultured using the cell mass-forming member according to an embodiment described above, and thus cells are likely to aggregate in the cell attachment region, making it easier to promote the formation of a collective cell mass with a three-dimensional structure.

In the method for producing cultured cells, cells are preferably seeded on the surface of the cell mass-forming member on which the cell attachment region and the attachment-preventing region are formed, and the cells are attached to the surface of the cell mass-forming member.

The cells are not particularly limited. Cells capable of forming a cell mass can be suitably selected. Examples include hepatocytes, osteoblasts, chondrocytes, myoblasts, fibroblasts, cardiomyocytes, blood immune cells, and vascular endothelial cells.

Stem cells with differentiation potential, such as iPS cells or ES cells, or differentiated cells derived from stem cells may be used as the cells. Alternatively, tumor cells may be used as the cells. The tumor cells are preferably epithelial tumor cells, and examples include cervical cancer cells, such as HeLa cells; pancreatic cancer cells; lung cancer cells; large bowel cancer cells; and head and neck cancer cells.

Animal species of the cells, stem cells, differentiated cells, and tumor cells are not particularly limited. Examples include mammals, such as humans, pigs, mice, rats, rabbits, guinea pigs, hamsters, cattle, horses, cats, dogs, sheep, and goats.

The culture conditions are not particularly limited and can be changed according to the cell type, the use of the cell mass, and/or the like.

When confluent cultured cells are detached, cell detachment agent may be used. The cell detachment agent is any cell detachment agent commonly used in cell culture and is not particularly limited.

### Cultured cells with cell mass-forming member

Cultured cells with a cell mass-forming member according to an embodiment includes: the cell mass-forming member according to an embodiment; and cultured cells attached to the cell attachment region of the cell mass-forming member.

Cultured cells with a cell mass-forming member according to an embodiment is produced by using the cell mass-forming member described above and culturing cells in a state where the cells are attached to the surface of the cell mass-forming member. Thus, a cell mass with any desired three-dimensional structure containing cultured cells can attach to the surface of the cell mass-forming member.

The type of cell is not particularly limited, and examples include the same cells as those exemplified in the section of the method for producing cultured cells. The cultured cells with a cell mass-forming member can be suitably applied to regenerative medicine, research on viruses and bacterial infections, and methods for screening therapeutic agents for diseases. When the cultured cells with a cell mass-forming member is applied to the development of a therapeutic agent for disease, the agent is one that exerts either a preventive effect or a therapeutic effect on the disease or both effects.

In the screening method, a cell mass containing target cells of disease or tumor cells is allowed to coexist with a test substance, and the presence or absence of attenuation or loss of a morphology specific to the target cells is observed in the coexistence of the test substance. Then, the test substance is determined on whether it has a preventive effect or a therapeutic effect on the disease.

Although an embodiment has been described above, the present invention is not limited to the embodiments disclosed in the present specification and can be appropriately modified and implemented without changing the gist of the present invention. The embodiments disclosed in the present specification can be implemented in a variety of other forms, and various omissions, substitutions, and changes can be made without departing from the gist of the invention.

### Examples

Hereinafter, an embodiment will be described in further detail using examples; however, the present invention is not limited to these examples.

### Example 1

A single particle film etching mask made of colloidal silica was prepared by the method described in JP 2009-034630 A on a Si wafer with a spot pattern (photoresist) obtained using a typical photolithography method. A fine uneven structure was formed on the Si substrate by a vapor phase etching method using this single particle film etching mask. The remaining photoresist was removed, and a protruded Si-surface fine-structure original plate (1) in which 100 circular and spot-like fine uneven structure regions with a diameter of 30 µm were arranged at intervals of 40 µm at the shortest per mm² was prepared. In the protruded Si-surface fine structure original plate (1), a plurality of substantially conical protrusions was included in the fine uneven structure region, in which the average pitch between the plurality of protrusions was 200 nm and the average height of the plurality of protrusions was 530 nm. In addition, the height difference h between the tip of the protrusion of the circular and spot-like fine uneven structure region and the flat region outside the fine uneven structure region was 270 nm.

Then, a recessed Ni-electroformed stamper (1) was prepared from the protruded Si-surface fine structure original plate (1). Thereafter, a protruded structure serving as a carrying part was transferred to a surface of a cycloolefin polymer film with a thickness of 188 µm by a thermal nanoimprint method using the recessed Ni-electroformed stamper (1), and a protruded resin transfer member (1) was prepared. Specifically, the fine structure surface of the recessed Ni-electroformed stamper (1) disposed to face the surface of a cycloolefin polymer film with a thickness of 188 µm was set in a pressing unit of a nanoimprinter. Temperature increase was started in this state, and pressure was applied at 5 MPa at 175°C for 5 minutes. After a lapse of 5 minutes, the pressing unit was cooled to room temperature, and the recessed Ni-electroformed stamper (1) was released from the surface of the cycloolefin polymer film, and the protruded resin transfer member (1) was obtained. This protruded resin transfer member (1) was used as the protruded cell mass-forming member of Example 1.

### Example 2

A protruded structure serving as a carrying part was transferred from the protruded Si-surface fine structure original plate (1) prepared in Example 1 to a surface of a cycloolefin polymer film with a thickness of 188 µm by a thermal nanoimprint method, and a recessed resin original plate (1) was prepared. A recessed cell mass-forming member was obtained in the same manner as in Example 1 except that a protruded Ni-electroformed stamper (1) was prepared from the recessed resin original plate (1) and the protruded Ni-electroformed stamper (1) was used.

### Example 3

A single particle film etching mask made of colloidal silica was prepared by the method described in JP 2009-034630 A on a Si wafer with a ring pattern (photoresist) obtained using a typical photolithography method. An annular fine uneven structure region was formed on the Si substrate by a vapor phase etching method using this single particle film etching mask (the remaining photoresist was removed). The annular fine uneven structure had a diameter R of the outer diameter circle of 40 µm, a diameter r of the inner diameter circle of 30 µm, and a width W of 5 µm. A protruded Si-surface fine-structure original plate (2) in which 100 annular fine uneven structure regions were arranged at intervals of 30 µm at the shortest per mm² was prepared. In the protruded Si-surface fine structure original plate (2), a plurality of substantially conical protrusions was included in the annular fine uneven structure region, in which the average pitch between the plurality of protrusions was 400 nm and the average height of the plurality of protrusions was 1100 nm. The height difference h between the tip of the protrusion of the annular fine uneven structure region and the flat region outside the fine uneven structure region was 500 nm.

Thereafter, a protruded cell mass-forming member was obtained in the same manner as in Example 1 except that a recessed Ni-electroformed stamper (2) prepared from the protruded Si-surface fine structure original plate (2) was used.

### Example 4

A protruded structure serving as a carrying part was transferred from the protruded Si-surface fine structure original plate (2) prepared in Example 3 to a surface of a cycloolefin polymer film with a thickness of 188 µm by a thermal nanoimprint method, and a recessed resin original plate (2) made of the cycloolefin polymer was prepared. A recessed cell mass-forming member was obtained in the same manner as in Example 1 except that a protruded Ni-electroformed stamper (2) prepared from the recessed resin original plate (2) was used.

### Culture of human pancreatic ductal adenocarcinoma cell line PCI-55

The surface of each of the cell mass-forming members obtained in Examples 1 to 4 was subjected to hydrophilization treatment by a UV ozone method, then coated with an MPC polymer solution by spin coating, and a coating layer of the MPC polymer with an average thickness of 40 nm was provided on the surface of the substrate. The coating layer was dried, and a cell mass-forming member with an attachment-preventing region and a cell attachment region formed on the surface was obtained. The cell mass-forming member was washed with distilled water, the air-dried cell mass-forming member was cut into a size of 20 mm × 20 mm, and was fixed with petrolatum to the inner bottom of a culture Petri dish with a diameter of 40 mm × a depth of 13.5 mm (Asnol Petri Dish, AS ONE Corporation). A DMEM (Dulbecco's Modified Eagle Medium) was then added to wash the cell mass-forming member three times, then 2 × 10⁶ PCI-55 cells suspended in 3 mL of a DMEM were seeded on the cell mass-forming member of each example and cultured at 37°C overnight to 48 hours.

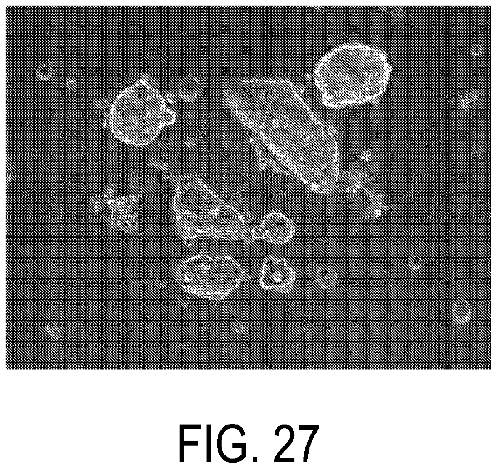

FIG. 27 is a DIC observation image showing the result of culturing the human pancreatic ductal adenocarcinoma cell line PCI-55 after applying the MPC polymer to the cell mass-forming member of Example 1. A plurality of polypoid cell masses each extending over two or more of the cell attachment regions and firmly adhered to the cell attachment regions was formed.

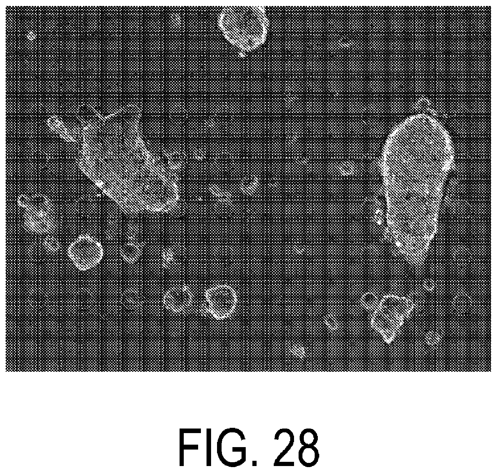

FIG. 28 is a DIC observation image showing the result of culturing the human pancreatic ductal adenocarcinoma cell line PCI-55 after applying the MPC polymer to the cell mass-forming member of Example 2. A plurality of polypoid cell masses each extending in the height direction of one cell attachment region was formed.

The above culture results confirmed formation of self-assembled anchorage-dependent non-spheroid cancer cell masses with morphological polarity and tissue movement polarity in the cell mass-forming members of Examples 1 to 4.

### Culture of mouse myoblast C2C12 line

An MPC polymer solution was applied to the entire surface of each of the cell mass-forming members obtained in Examples 1 to 4 by spin coating, and a coating layer of the MPC polymer with an average thicknesses 40 nm was provided on the surface of the substrate. The coating layer was dried, and a cell mass-forming member with an attachment-preventing region and a cell attachment region formed on the surface was obtained. The cell mass-forming member was washed with distilled water, the air-dried cell mass-forming member was cut into a size of 20 mm x 20 mm, and was fixed with petrolatum to the inner bottom of a culture Petri dish with a diameter of 40 mm × a depth of 13.5 mm (Asnol Petri Dish, AS ONE Corporation). A DMEM was added to wash the cell mass-forming member three times, then 5 × 10⁵ C2C12 cells suspended in 3 mL of a DMEM containing 10% FBS were seeded on each cell mass-forming member and cultured at 37°C overnight to 48 hours.

After completion of the culture, cell masses were fixed with paraformaldehyde, actin was stained with phalloidin, cell nuclei were stained with DAPI, and a two dimensional image was obtained with an all-in-one fluorescence microscope (BZ-X800, Keyence Corporation).

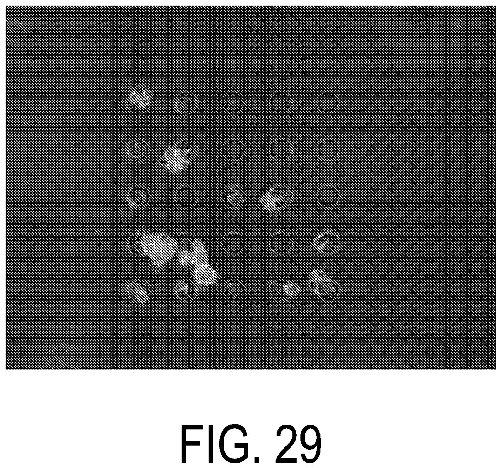

FIG. 29 is a DIC observation image showing the result of culturing the mouse myoblast C2C12 line using the cell mass-forming member of Example 1. Formation of non-spheroid cell masses each extending over two or more spots was observed.

Similarly, also when the mouse myoblast cell line C2C12 was cultured using each of the cell mass-forming members of Examples 2 to 4, formation of non-spheroid cell masses each extending over two or more spots was observed.

The above culture results confirmed formation of self-assembled anchorage-dependent non-spheroid cell masses with morphological polarity and tissue movement polarity in the cell mass-forming members of Examples 1 to 4.

### Industrial Applicability

According to an embodiment, there are provided the cell mass-forming member that can easily form a cell mass and has excellent industrial mass productivity; the culture vessel including the cell mass-forming member; the method for producing cultured cells using the cell mass-forming member; and the cultured cells with the cell mass-forming member including the cell mass-forming member.

### Reference Signs List

1 Cell mass-forming member, 2 Substrate, 3 Attachment-preventing region, 4 Cell attachment region, 5 Hydrophilic coating layer, 6 Fine uneven structure region, 7 Protrusion, 8 Hydrophilic layer, 9, 10 Cell-forming region, 11 Recess

## Claims

1. A cell mass-forming member comprising a substrate, wherein
on a surface of the substrate, at least one or more attachment-preventing regions and at least one or more cell attachment regions are formed,
in at least a part of the at least one or more cell attachment regions, a fine uneven structure region comprising a plurality of protrusions or a plurality of recesses is formed, and
in both the at least one or more attachment-preventing regions and the at least one or more cell attachment regions, a hydrophilic coating layer is formed.

2. A cell mass-forming member comprising a substrate, wherein
on a surface of the substrate, at least one or more attachment-preventing regions and at least one or more cell attachment regions are formed,
in at least a part of the at least one or more cell attachment regions, a fine uneven structure region comprising a plurality of protrusions or a plurality of recesses is formed, and
in either the at least one or more attachment-preventing regions or the at least one or more cell attachment regions, a hydrophilic coating layer is formed.

3. The cell mass-forming member according to claim 1 or 2, wherein an average height of the plurality of protrusions or an average depth of the plurality of recesses is 250 nm or greater.

4. The cell mass-forming member according to any one of claims 1 to 3, wherein an aspect ratio of the average height of the plurality of protrusions to an average diameter of the plurality of protrusions, or an aspect ratio of the average depth of the plurality of recesses to an average diameter of the plurality of recesses is from 0.5 to 5.0.

5. The cell mass-forming member according to any one of claims 1 to 4, wherein an average pitch between the plurality of protrusions or between the plurality of recesses is from 50 nm to 1 µm.

6. The cell mass-forming member according to any one of claims 1 to 5, wherein the at least one or more cell attachment regions are located at a position with a height difference relative to the at least one or more attachment-preventing regions in a thickness direction of the substrate.

7. The cell mass-forming member according to any one of claims 1 to 5, wherein a height difference between the at least one or more cell attachment regions and the surface of the substrate is from 0 to 3 µm.

8. The cell mass-forming member according to any one of claims 1 to 7, wherein a hydrophilic layer is formed in recesses between the plurality of protrusions of the fine uneven structure region or in bottoms of recesses between the plurality of protrusions of the fine uneven structure region.

9. The cell mass-forming member according to any one of claims 1 to 8, wherein the substrate comprises at least one or more resins selected from the group consisting of polyethylene terephthalate), triacetyl cellulose, polycarbonate, cycloolefin polymers, cycloolefin copolymers, acrylic resins, polystyrene, and dimethylpolysiloxane.

10. The cell mass-forming member according to any one of claims 1 to 9, wherein a ratio of a major axis of an ellipse with a minimum area circumscribing the at least one or more cell attachment regions to a minor axis of the ellipse is from 1 to 3.

11. The cell mass-forming member according to claim 1, wherein
in a plan view of the cell mass-forming member, the at least one or more cell attachment regions are formed in an annular region surrounded by an outer diameter circle and an inner diameter circle, the outer-diameter circle having a diameter from 10 to 100 µm, a difference between the diameter of the outer diameter circle and a diameter of the inner diameter circle being from 200 nm to 30 µm, and in at least a part of the annular region, a fine uneven structure comprising a plurality of protrusions or a plurality of recesses being formed, and
in both the at least one or more attachment-preventing regions and the at least one or more cell attachment regions, a hydrophilic coating layer is formed.

12. A culture vessel comprising the cell mass-forming member described in any one of claims 1 to 11.

13. A method for producing cultured cells, the method comprising culturing cells using the cell mass-forming member described in any one of claims 1 to 11, and then detaching the cultured cells from the cell mass-forming member.

14. Cultured cells with a cell mass-forming member comprising:
the cell mass-forming member described in any one of claims 1 to 11; and
cultured cells attached to the cell mass-forming member.

15. The cultured cells with a cell mass-forming member according to claim 14, wherein a cell mass comprising the cells forms a three-dimensional structure.
